# EUROPÄISCHE PATENTSCHRIFT

(11) **EP 1 423 528 B1**
(45) Veröffentlichungstag und Bekanntmachung des Hinweises auf die Patenterteilung: **02.01.2008**
(21) Anmeldenummer: 01980315.4
(22) Anmeldetag: 01.09.2001
(51) Int. Cl.: C12Q 1/68

(54) **VERFAHREN ZUR BESTIMMUNG DES METHYLIERUNGSGRADES VON BESTIMMTEN CYTOSINEN IN GENOMISCHER DNA IM SEQUENZKONTEXT 5'-CPG-3'**
METHOD FOR DETERMINING THE DEGREE OF METHYLATION OF DEFINED CYTOSINES IN GENOMIC DNA IN THE SEQUENCE CONTEXT 5'-CPG-3'
PROCEDE DE DETERMINATION DU DEGRE DE METHYLATION DE CYTOSINES DETERMINEES D'ADN GENOMIQUE DANS LE CONTEXTE SEQUENTIEL 5'-CPG-3'

(30) Priorität: 01.09.2000 DE 10043826; 05.09.2000 DE 10044543
(43) Veröffentlichungstag der Anmeldung: 02.06.2004
(60) Teilanmeldung: 07119103.5
(73) Patentinhaber: Epigenomics AG, 10178 Berlin (DE)
(72) Erfinder: OLEK, Alexander, 10115 Berlin (DE); PIEPENBROCK, Christian, 10115 Berlin (DE); BERLIN, Kurt, 14532 Stahnsdorf (DE); GÜTIG, David, 10435 Berlin (DE)
(74) Vertreter: Schubert, Klemens
(86) Internationale Anmeldenummer: PCT/EP2001/010074
(87) Internationale Veröffentlichungsnummer: WO 2002/018632

(56) Entgegenhaltungen:
- WO-A-95/11995
- WO-A-99/28498
- DATABASE EMBL [Online] Human flotillin cDNA 23. September 1998 (1998-09-23), XP002230584 accession no. EMBL Database accession no. AF089750
- NIEMEYER C M ET AL: "DNA MICROARRAYS**" ANGEWANDTE CHEMIE, VCH VERLAGSGESELLSCHAFT, WEINHEIM, DE, Bd. 38, Nr. 19, 1999, Seiten 3039-3043, XP000961724 ISSN: 0044-8249
- REIN ET AL: "Identifying 5-methylcytosine and related modifications in DNA genomes" NUCLEIC ACIDS RESEARCH, OXFORD UNIVERSITY PRESS, SURREY, GB, Bd. 26, Nr. 10, 1998, Seiten 2255-2264, XP002143106 ISSN: 0305-1048
- HERMAN J G ET AL: "METHYLATION-SPECIFIC PCR: A NOVEL PCR ASSAY FOR METHYLATION STATUS OF CPG ISLANDS" PROCEEDINGS OF THE NATIONAL ACADEMY OF SCIENCES OF USA, NATIONAL ACADEMY OF SCIENCE. WASHINGTON, US, Bd. 93, 1. September 1996 (1996-09-01), Seiten 9821-9826, XP002910406 ISSN: 0027-8424
- VOLONTE D ET AL: "Flotillins/cavatellins are differentially expressed in cells and tissues and form hetero-oligomeric complex with caveolins in vivo" JOURNAL OF BIOLOGICAL CHEMISTRY, AMERICAN SOCIETY OF BIOLOGICAL CHEMISTS, BALTIMORE, MD, US, Bd. 274, Nr. 18, 30. April 1999 (1999-04-30), Seiten 12702-12709, XP002171909 ISSN: 0021-9258

## Beschreibung

Die Erfindung betrifft ein Verfahren zur Detektion des Methylierungsgrades eines bestimmten Cytosins im Sequenzkontext 5'-CpG-3' einer genomischen DNA-Probe.

Die nach den methodischen Entwicklungen der letzten Jahre in der Molekularbiologie gut studierten Beobachtungsebenen sind die Gene selbst, die Übersetzung dieser Gene in RNA und die daraus entstehenden Proteine. Wann im Laufe der Entwicklung eines Individuums welches Gen angeschaltet wird und wie Aktivieren und Inhibieren bestimmter Gene in bestimmten Zellen und Geweben gesteuert wird, ist mit Ausmaß und Charakter der Methylierung der Gene bzw. des Genoms korrelierbar. Insofern äußern sich pathogene Zustände in einem veränderten Methylierungsmuster einzelner Gene oder des Genoms.

Die vorliegende Erfindung beschreibt ein Verfahren, mit dem viele Cytosinbasen in einer gegebenen DNA-Probe gleichzeitig auf das Vorliegen einer Methylgruppe an der Position 5 mittels Hybridisierung untersucht werden können. Sie beschreibt weiterhin Nukleinsäuren, Oligonukleotide und PNA-Oligomere welche nützlich sind um das Verfahren zur Diagnose von bestehenden Erkrankungen oder der Prädisposition für Erkrankungen einzusetzen.

5-Methylcytosin ist die häufigste kovalent modifizierte Base in der DNA eukaryotischer Zellen. Sie spielt beispielsweise eine Rolle in der Regulation der Transkription, beim genetischen Imprinting und in der Tumorgenese. Die Identifizierung von 5-Methylcytosin als Bestandteil genetischer Information ist daher von erheblichem Interesse. 5-Methylcytosin-Positionen können jedoch nicht durch Sequenzierung identifiziert werden, da 5-Methylcytosin das gleiche Basenpaarungsverhalten aufweist wie Cytosin. Darüber hinaus geht bei einer PCR-Amplifikation die epigenetische Information, welche die 5-Methylcytosine tragen, vollständig verloren.

Die Methylierung von CpG Inseln wird oft mit Transkriptionsinaktivität gleichgesetzt. Obwohl es eindeutige Beweise dafür gibt, das die CpG Inseln in den Promotoren der Gene zu finden sind, sind nicht alle CpG Inseln und Methylierungsstellen in bekannten Promotoren lokalisiert. Bei verschiedenen gewebsspezifischen und "imprinting" Genen sind die CpG Inseln in beträchtlichen Entfernungen stromabwärts des Transkriptionsstarts lokalisiert, zusätzlich besitzen viele Gene multiple Promotoren. Für eine Vielzahl von Erkrankungen wurde Methylierung von CpG Dinukleotiden als ursächlich nachgewiesen. Im Gegensatz zu klassischen Mutationen, handelt es sich bei der DNA-Methylierung um einen Mechanismus, der eine Basensubstitution beschreibt, ohne die codierende Funktion eines Gens zu verändern. Dieses Wechselspiel zwischen epigenetischer Modifikation und klassischen Mutationen spielt eine wichtige Rolle in der Tumorgenese. Beispielsweise sind fokale Hypermethylierung und generalisierte genomische Demethylierung Merkmale vieler verschiedener Tumortypen. Es wird angenommen, dass die Tumorgenese und die Tumorprogression zum einen durch Hypermethylierung induzierter Mutationsereignisse und zum anderen durch das Abschalten von Genen, welche die zelluläre Proliferation und/oder die induzierte Reaktivierung von Genen über Demethylierung kontrollieren, die nur für die embryologische Entwicklung gebraucht werden, verursacht werden.

Bei dem vererbbaren non-polyposis Colorektalkrebs beruht z. B. der Hauptteil der mutationsnegativen Dickdarmkrebs Fälle eher auf der Hypermethylierung des hMLH1 Promotors und der anschließenden Nicht-Expression von hMLH1, ein Reparaturgen für Fehlpaarungen (Bevilacqua RA, Simpson AJ. Methylation of the hMLH1 promoter but no hMLH1 mutations in sporadic gastric carcinomas with high-level microsatellite instability. Int J Cancer. 2000 Jul 15;87(2):200-3.). Bei der Pathogenese von Lungenkrebs ist der Verlust der Expression mit der Methylierung von CpG Inseln in der Promotor Sequenz eines RAS Effektor-Homologs korreliert. (Dammann R, Li C, Yoon JH, Chin PL, Bates S, Pfeifer GP, Nucleotide. Epigenetic inactivation of a RAS association domain family protein from the lung tumour suppressor locus3p21.3. Nat Genet. 2000 Jul; 25(3):315-9). Eine epigenetische Inaktivierung des LKB1 Tumorsupressorgens, welche die Hypermethylierung des Promotors mit einschliesst, ist mit dem Peutz-Jeghers Syndrom assoziiert (Esteller M, Avizienyte E, Corn PG, Lothe RA, Baylin SB, Aaltonen LA, Herman JG, Epigenetic inactivation of LKB1 in primary tumors associated with the Peutz-Jeghers syndrome. Oncogene. 2000 Jan 6;19(1):164-8).

Eine Vielzahl von Erkrankungen, die mit Methylierung assoziiert sind, weisen in ihrer Ätiologie eine enge Verbindung zu den Tumorsupressorgenen p16 oder p15 Gene auf. So wird eine Beziehung zwischen Mycoso fungoides und der Hypermethylierung des p16(INK4a) Gens angenommen (Navas IC, Ortiz-Romero PL, Villuendas R, Martinez P, Garcia C, Gomez E, Rodriguez JL, Garcia D, Vanaclocha F, Iglesias L, Piris MA, Algara P, p16 (INK4a) gene alterations are frequent in lesions of mycosis fungoides. Am J Pathol. 2000 May; 156(5):1565-72). Auch wird von einer starken Korrelation zwischen dem Abschalten der Transkription des p16 Gens bei dem gastrischen Karzinom und der de novo Methylierung einiger weniger spezifischer CpG Stellen ausgegangen (Song SH, Jong HS, Choi HH, Kang SH, Ryu MH, Kim NK, Kim WH, Bang YJ, Methylation of specific CpG sites in the promoter region could significantly down-regulate p16(INK4a) expression in gastric adenocarcinoma. Int J Cancer. 2000 Jul 15;87(2):236-40). Die Pathogenese des Cholangiokarzinoms, welches mit der primär sklerosierenden Cholangitis assoziiert ist, wird mit der Inaktivierung des p16 Tumorsupressorgens, welches wiederum von der Methylierung des p16 Promotors abhängig ist, in Zusammenhang gebracht (Ahrendt SA, Eisenberger CF, Yip L, Rashid A, Chow JT, Pitt HA, Sidransky D, Chromosome 9p21 loss and p16 inactivation in primary sclerosing cholangitis-associated cholangiocarcinoma. J Surg Res. 1999 Jun 1;84(1):88-93). Bei der Leukämogenese und beim Fortschreiten der akuten lymphatischen Leukämie spielt die Inaktivierung des p16 Gens durch Hypermethylierung eine Rolle (Nakamura M, Sugita K, Inukai T, Goi K, Iijima K, Tezuka T, Kojika S, Shiraishi K, Miyamoto N, Karakida N, Kagami K, O-Koyama T, Mori T, Nakazawa S, p16/MTS1/INK4A gene is frequently inactivated by hypermethylation in childhood acute lymphoblastic leukemia with 11q23 translocation. Leukemia. 1999 Jun; 13(6):884-90). Weiterhin wird postuliert, dass die Hypermethylierung der p16 und p15 Gene eine entscheidende Rolle bei der Tumorgenese des multiplen Myeloms spielt (Ng MH, Wong IH, Lo KW, DNA methylation changes and multiple myeloma. Leuk Lymphoma. 1999 Aug;34(5-6):463-72). An der Prädisposition des Nierenkarzinoms scheint das durch Methylierung inaktivierte VHL-Gen beteiligt zu sein (Glavac D, Ravnik-Glavac M, Ovcak Z, Masera A, Genetic changes in the origin and development of renal cell carcinoma (RCC). Pflugers Arch. 1996;431(6 Suppl 2):R193-4). Eine abweichende Methylierung der 5' CpG Insel ist möglicherweise an der Transkriptionsinaktivierung des p16 Gens bei dem nasopharyngealen Karzinom beteiligt (Lo KW, Cheung ST, Leung SF, van Hasselt A, Tsang YS, Mak KF, Chung YF, Woo JK, Lee JC, Huang DP, Hypermethylation of the p16 gene in nasopharyngeal carcinoma. Cancer Res. 1996 Jun 15;56(12):2721-5). Bei dem Leberzellenkarzinom wurde eine Inaktivierung des p16 Proteins nachgewiesen. Promotor Hypermethylierung und homozygote Deletionen gehören hier zu den häufigen Mechanismen (Jin M, Piao Z, Kim NG, Park C, Shin EC, Park JH, Jung HJ, Kim CG, Kim H, p16 is a major inactivation target in hepatocellular carcinoma. Cancer. 2000 Jul 1;89(1):60-8). DNA-Methylierung als Kontrolle der Genexpression wurde für das BRCA1 Gen für Brustkrebs nachgewiesen (Magdinier F, Billard LM, Wittmann G, Frappart L, Benchaib M, Lenoir GM, Guerin JF, Dante R Regional methylation of the 5' end CpG island of BRCA1 is associated with reduced gene expression in human somatic cells FASEB J. 2000 Aug;14(11):1585-94). Eine Korrelation zwischen Methylierung und Non-Hodgekin's Lymphom wird ebenfalls angenommen (Martinez-Delgado B, Richart A, Garcia MJ, Robledo M, Osorio A, Cebrian A, Rivas C, Benitez J, Hypermethylation of P16ink4a and P15ink4b genes as a marker of disease in the follow-up of non-Hodgkin's lymphomas. Br J Haematol. 2000 Apr;109(1):97-103).

CpG-Methylierung ruft auch das Fortschreiten der T-Zell Leukämie hervor, die in Zusammenhang mit der verminderten Expression des CDKN2A Gens steht (Nosaka K, Maeda M, Tamiya S, Sakai T, Mitsuya H, Matsuoka M, Increasing methylation of the CDKN2A gene is associated with the progression of adult T-cell leukemia. Cancer Res. 2000 Feb 15;60(4):1043-8). Bei Blasenkrebs wurde eine erhöhte Methylierung der CpG-Inseln festgestellt (Salem C, Liang G, Tsai YC, Coulter J, Knowles MA, Feng AC, Groshen S, Nichols PW, Jones PA, Progressive increases in de novo methylation of CpG islands in bladder cancer. Cancer Res. 2000 May 1;60(9):2473-6). Die Transkriptionsinaktivierung von Plattenepithelkarzinomen der Speiseröhre wird in Verbindung mit der Methylierung des FHIT Gens gebracht, das mit dem Fortschreiten der Erkrankung assoziiert ist (Shimada Y, Sato F, Watanabe G, Yamasaki S, Kato M, Maeda M, Imamura M, Loss of fragile histidine triad gene expression is associated with progression of esophageal squamous cell carcinoma, but not with the patient's prognosis and smoking history. Cancer. 2000 Jul 1;89(1):5-11). Die Neutral Endopeptidase 24.11 (NEP) inaktiviert das Wachstum von Neuropeptipen, die an dem Wachstum des Androgen unabhängigen Prostatakrebs beteiligt sind. Ein Verlust der NEP Expression durch Hypermethylierung des NEP-Promotors trägt möglicherweise zu der Entwicklung des Neuropeptid stimulierten Androgen unabhängigen Prostatakrebses bei (Usmani BA, Shen R, Janeczko M, Papandreou CN, Lee WH, Nelson WG, Nelson JB, Nanus DM, Methylation of the neutral endopeptidase gene promoter in human prostate cancers. Clin Cancer Res. 2000 May;6(5):1664-70). Der adrenokortikale Tumor bei Erwachsenen weist strukturelle Abnormalitäten bei Tumor-DNA auf. Diese Abnormalitäten beinhalten unter anderem eine Überexpression des IGF2 Gens in Korrelation mit einer Demethylierung der DNA an diesem Locus (Wilkin F, Gagne N, Paquette J, Oligny LL, Deal C, Pediatric adrenocortical tumors: molecular events leading to insulin-like growth factor II gene overexpression. J Clin Endocrinol Metab. 2000 May;85(5):2048-56. Review). Es wird angenommen, dass bei dem Retinoblastomgen DNA-Methylierungen in mehreren Exons an der Erkrankung beteiligt sind (Mancini D, Singh S, Ainsworth P, Rodenhiser D, Constitutively methylated CpG dinucleotides as mutation hot spots in the retinoblastoma gene (RB1). Am J Hum Genet. 1997 Jul;61(1):80-7). Bei chronischer myeloischer Leukämie wird ein Zusammenhang zwischen der Deregulation des p53 Gens und einer Veränderung des Methylierungsmusters mit der Progression der Erkrankung vermutet (Guinn BA, Mills KI, p53 mutations, methylation and genomic instability in the progression of chronic myeloid leukaemia. Leuk Lymphoma. 1997 Jul;26(3-4):211-26). Auch für die akute myeloische Leukämie wurde ein Zusammenhang mit Methylierung nachgewiesen (Melki JR, Vincent PC, Clark SJ. Concurrent DNA hypermethylation of multiple genes in acute myeloid leukemia. Cancer Res. 1999 Aug 1;59(15):3730-40). Es wurde eine tumorspezifische Methylierungsstelle in dem Supressorgen für den Wilms Tumor identifiziert (Kleymenova EV, Yuan X, LaBate ME, Walker CL, Identification of a tumor-specific methylation site in the Wilms tumor suppressor gene. Oncogene. 1998 Feb 12;16(6):713-20). Bei dem Burkitt Lymphom weisen einige Promotoren eine vollständige CpG-Methylierung auf (Tao Q, Robertson KD, Manns A, Hildesheim A, Ambinder RF, Epstein-Barr virus (EBV) in endemic Burkitt's lymphoma: molecular analysis of primary tumor tissue. Blood. 1998 Feb 15;91(4):1373-81). Es wird angenommen, dass bei dem Schilddrüsenkarzinom die DNA-Methylierung eine Rolle spielt (Venkataraman GM, Yatin M, Marcinek R, Ain KB, Restoration of iodide uptake in dedifferentiated thyroid carcinoma: relationship to human Na+/I-symporter gene methylation status. J Clin Endocrinol Metab. 1999 Jul ; 84 (7) : 2449-57).

Nicht nur viele Krebserkrankungen sind mit Methylierung assoziiert, auch viele nicht Krebserkrankungen werden mit Methylierung in Zusammenhang gebracht. So weisen Untersuchungen zur entzündlichen Arthritis darauf hin, dass diese Erkrankung mit einer Untermethylierung genomischer DNA assoziiert ist (Kim YI, Logan JW, Mason JB, Roubenoff R, DNA hypomethylation in inflammatory arthritis: reversal with methotrexate. J Lab Clin Med. 1996 Aug;128(2):165-72). Für das ICF-Syndrom wurde eine Methylierungs regulierte Expression nachgewiesen (Kondo T, Bobek MP, Kuick R, Lamb B, Zhu X, Narayan A, Bourc'his D, Viegas-Pequignot E, Ehrlich M, Hanash SM, Whole-genome methylation scan in ICF syndrome: hypomethylation of nonsatellite DNA repeats D4Z4 and NBL2). Die Beteiligung von Methylierung an systemischem Lupus erythematosus wird vermutet (Vallin H, Perers A, Alm GV, Ronnblom L, Antidouble-stranded DNA antibodies and immunostimulatory plasmid DNA in combination mimic the endogenous IFN-alpha inducer in systemic lupus erythematosus. J Immunol. 1999 Dec1;163(11):6306-13); ebenso ein Zusammenhang zwischen der Muskeldystrophy Duchenne und einer CpG reichen Insel (Banerjee S, Singh PB, Rasberry C, Cattanach BM, Embryonic inheritance of the chromatin organisation of the imprinted H19 domain in mouse spermatozoa. Mech Dev. 2000 Feb;90(2):217-26; Burmeister M, Lehrach H, Longrange restriction map around the Duchenne muscular dystrophy gene. Nature. 1986 Dec 11-17;324(6097):582-5). Ein epigenetischer Effekt, der an der Untermethylierung des Amyloid Precursor Proteins, beteiligt ist, welches mit der Ausbildung der Erkrankung in Zusammenhang steht, wird bei der Alzheimer Erkrankung vermutet (West RL, Lee JM, Maroun LE, Hypomethylation of the amyloid precursor protein gene in the brain of an Alzheimer's disease patient. J Mol Neurosci. 1995;6(2):141-6). Auch auf chromosomaler Ebene spielt der Methylierungsstatus eine wichtige Rolle. Beispielsweise wird bei mentalen Retardierungssyndromen, die mit der Fragilität des X-Chromosoms gekoppelt sind, der Grad der Chromosomenbrüchigkeit durch die Methylierung bestimmt (de Muniain AL, Cobo AM, Poza JJ, Saenz A, [Diseases due to instability of DNA]. Neurologia. 1995 Dec;10 Suppl 1:12-9).

Eine relativ neue und die mittlerweile am häufigsten angewandte Methode zur Untersuchung von DNA auf 5-Methylcytosin beruht auf der spezifischen Reaktion von Bisulfit mit Cytosin, das nach anschließender alkalischer Hydrolyse in Uracil umgewandelt wird, welches in seinem Basenpaarungsverhalten dem Thymidin entspricht. 5-Methylcytosin wird dagegen unter diesen Bedingungen nicht modifiziert. Damit wird die ursprüngliche DNA so umgewandelt, dass Methylcytosin, welches ursprünglich durch sein Hybridisierungsverhalten vom Cytosin nicht unterschieden werden kann, jetzt durch "normale" molekularbiologische Techniken als einzig verbliebenes Cytosin beispielsweise durch Amplifikation und Hybridisierung oder Sequenzierung nachgewiesen werden kann. Alle diese Techniken beruhen auf Basenpaarung, welche jetzt voll ausgenutzt wird. Der Stand der Technik, was die Empfindlichkeit betrifft, wird durch ein Verfahren definiert, welches die zu untersuchende DNA in einer Agarose-Matrix einschließt, dadurch die Diffusion und Renaturierung der DNA (Bisulfit reagiert nur an einzelsträngiger DNA) verhindert und alle Fällungs- und Reinigungsschritte durch schnelle Dialyse ersetzt (Olek, A. et al., Nucl. Acids. Res. 1996, 24, 5064-5066). Mit dieser Methode können einzelne Zellen untersucht werden, was das Potential der Methode veranschaulicht. Allerdings werden bisher nur einzelne Regionen bis etwa 3000 Basenpaare Länge untersucht, eine globale Untersuchung von Zellen auf Tausenden von möglichen Methylierungsanalysen ist nicht möglich. Allerdings kann auch dieses Verfahren keine sehr kleinen Fragmente aus geringen Probenmengen zuverlässig analysieren. Diese gehen trotz Diffusionsschutz durch die Matrix verloren.

Eine Übersicht über die weiteren bekannten Möglichkeiten, 5-Methylcytosine nachzuweisen, kann aus dem folgenden Übersichtsartikel entnommen werden: Rein, T., DePamphilis, M. L., Zorbas, H., Nucleic Acids Res. 1998, 26, 2255.

Die Bisulfit-Technik wird bisher bis auf wenige Ausnahmen (z. B. Zechnigk, M. et al., Eur. J. Hum. Gen. 1997, 5, 94-98) nur in der Forschung angewendet. Immer aber werden kurze, spezifische Stücke eines bekannten Gens nach einer Bisulfit-Behandlung amplifziert und entweder komplett sequenziert (Olek, A. und Walter, J., Nat. Genet. 1997, 17, 275-276) oder einzelne Cytosin-Positionen durch eine "Primer-Extension-Reaktion" (Gonzalgo, M. L. und Jones, P. A., Nucl. Acids Res. 1997, 25, 2529-2531, WO-Patent 9500669) oder einen Enzymschnitt (Xiong, Z. und Laird, P. W., Nucl. Acids. Res. 1997, 25, 2532-2534) nachgewiesen. Zudem ist auch der Nachweis durch Hybridisierung beschrieben worden (Olek et al., WO-A 99/28498).

Weitere Publikationen, die sich mit der Anwendung der Bisulfit-Technik zum Methylierungsnachweis bei einzelnen Genen befassen, sind: Xiong, Z. und Laird, P. W. (1997), Nucl. Acids Res. 25, 2532; Gonzalgo, M. L. und Jones, P. A. (1997), Nucl. Acids Res. 25, 2529; Grigg, S. und Clark, S. (1994), Bioassays 16, 431; Zeschnik, M. et al. (1997), Human Molecular Genetics 6, 387; Teil, R. et al. (1994), Nucl. Acids Res. 22, 695; Martin, V. et al. (1995), Gene 157, 261; WO-A 97/46705, WO-A 95/15373 und WO-A 95/45560.

Eine Übersicht über den Stand der Technik in der Oligomer Array Herstellung läßt sich aus einer im Januar 1999 erschienenen Sonderausgabe von Nature Genetics (Nature Genetics Supplement, Volume 21, January 1999) und der dort zitierten Literatur entnehmen.

Für die Abtastung eines immobilisierten DNA-Arrays sind vielfach fluoreszenzmarkierte Sonden verwendet worden. Besonders geeignet für Fluoreszenzmarkierungen ist das einfache Anbringen von Cy3 und Cy5 Farbstoffen am 5'-OH der jeweiligen Sonde. Die Detektion der Fluoreszenz der hybridisierten Sonden erfolgt beispielsweise über ein Konfokalmikroskop. Die Farbstoffe Cy3 und Cy5 sind, neben vielen anderen, kommerziell erhältlich.

Matrix-assistierte Laser Desorptions/Ionisations-Massenspektrometrie (MALDI-TOF) ist eine sehr leistungsfähige Entwicklung für die Analyse von Biomolekülen (Karas, M. und Hillenkamp, F. (1988), Laser desorption ionization of proteins with molecular masses exeeding 10000 daltons. Anal. Chem. 60: 2299-2301). Ein Analyt wird in eine lichtabsorbierende Matrix eingebettet. Durch einen kurzen Laserpuls wird die Matrix verdampft und das Analytmolekül so unfragmentiert in die Gasphase befördert. Durch Stöße mit Matrixmolekülen wird die Ionisation des Analyten erreicht. Eine angelegte Spannung beschleunigt die Ionen in ein feldfreies Flugrohr. Auf Grund ihrer verschiedenen Massen werden Ionen unterschiedlich stark beschleunigt. Kleinere Ionen erreichen den Detektor früher als größere.

MALDI-TOF Spektrometrie eignet sich ausgezeichnet zur Analyse von Peptiden und Proteinen. Die Analyse von Nukleinsäuren ist etwas schwieriger (Gut, I. G. und Beck, S. (1995)), DNA and Matrix Assisted Laser Desorption Ionization Mass Spectrometry. Molecular Biology: Current Innovations and Future Trends 1: 147-157.) Für Nukleinsäuren ist die Empfindlichkeit etwa 100 mal schlechter als für Peptide und nimmt mit zunehmender Fragmentgröße überproportional ab. Für Nukleinsäuren, die ein vielfach negativ geladenes Rückgrat haben, ist der Ionisationsprozeß durch die Matrix wesentlich ineffizienter. In der MALDI-TOF Spektrometrie spielt die Wahl der Matrix eine eminent wichtige Rolle. Für die Desorption von Peptiden sind einige sehr leistungsfähige Matrices gefunden worden, die eine sehr feine Kristallisation ergeben. Für DNA gibt es zwar mittlererweile einige ansprechende Matrices, jedoch wurde dadurch der Empfindlichkeitsunterschied nicht verringert. Der Empfindlichkeitsunterschied kann verringert werden, indem die DNA chemisch so modifiziert wird, dass sie einem Peptid ähnlicher wird. Phosphorothioatnukleinsäuren, bei denen die gewöhnlichen Phosphate des Rückgrats durch Thiophosphate substituiert sind, lassen sich durch einfache Alkylierungschemie in eine ladungsneutrale DNA umwandeln (Gut, I. G. und Beck, S. (1995), A procedure for selective DNA alkylation and detection by mass spectrometry. Nucleic Acids Res. 23: 1367-1373). Die Kopplung eines "charge tags" an diese modifizierte DNA resultiert in der Steigerung der Empfindlichkeit um den gleichen Betrag, wie er für Peptide gefunden wird. Ein weiterer Vorteil von "charge tagging" ist die erhöhte Stabilität der Analyse gegen Verunreinigungen, die den Nachweis unmodifizierter Substrate stark erschweren.

Genomische DNA wird durch Standardmethoden aus DNA von Zell-, Gewebe- oder sonstigen Versuchsproben gewonnen. Diese Standardmethodik findet sich in Referenzen wie Fritsch und Maniatis eds., Molecular Cloning: A Laboratory Manual, 1989.

Die vorliegende Erfindung soll ein besonders effizientes und zuverlässiges Verfahren bereitstellen, das es erlaubt, viele Cytosinbasen in einer gegebenen DNA-Probe gleichzeitig auf das Vorliegen einer Methylgruppe an der Position 5 mittels Hybridisierung zu untersuchen. Dazu werden zudem Oligonukleotide und PNA-Oligomere bereitgestellt, welche für die Verwendung des Verfahrens zur Diagnose von bestehenden Erkrankungen und der Prädisposition für Erkrankungen durch Analyse eines Satzes genetischer und/oder epigenetischer Parameter besonders geeignet sind.

Insbesondere sind als Mutationen Insertionen, Deletionen, Punktmutationen, Inversionen und Polymorphismen und besonders bevorzugt SNPs (Single Nucleotide Polymorphisms) zu bezeichnen. Polymorphismen können aber ebenso Insertionen, Deletionen oder Inversionen sein.

Epigenetische Parameter im Sinne dieser Erfindung sind insbesondere Cytosin-Methylierungen und zu ihrer Regulation weiterhin erforderliche Sequenzen. Weitere epigenetische Parameter sind beispielsweise die Acetylierung von Histonen, die jedoch mit dem beschriebenen Verfahren nicht direkt analysiert werden kann, sondern wiederum mit der DNA-Methylierung korreliert.

Das vorliegende Verfahren dient zur Detektion des Methylierungsgrades mindestens eines bestimmten Cytosins im Sequenzkontext 5'-CpG-3' einer genomischen DNA-Probe. Besonders bevorzugt wird das Verfahren zur gleichzeitigen Detektion vieler verschiedener Methylierungspositionen verwendet.

Die Aufgabe wird erfindungsgemäß durch ein Verfahren zur Detektion des Methylierungsgrades eines bestimmten Cytosins im Sequenzkontext 5'-CpG-3' einer genomischen DNA-Probe gelöst, welches dadurch gekennzeichnet ist, dass
a) man die genomische DNA behandelt, wobei man die Cytosinbasen in Uracil umwandelt, nicht aber die 5-Methylcytosinbasen;
b) man definierte Fragmente amplifiziert, die das besagte bestimmte Cytosin enthalten, wobei die Amplifikate eine nachweisbare Markierung erhalten;
c) man die Amplifikate an zwei Klassen von Oligonukleotiden und/oder PNA-Oligomeren mit jeweils einem Mitglied für das besagte bestimmte Cytosin hybridisiert;
d) man das Ausmaß der Hybridisierung der Amplifikate an die zwei Klassen von Oligonukleotiden durch Detektion der Markierung der Amplifikate bestimmt;
e) man aus dem Verhältnis der an den zwei Klassen von Oligonukleotiden in Folge der Hybridisierung detektierten Markierungen auf den Methylierungsgrad besagten bestimmten Cytosins in der genomischen DNA-Probe schliesst.

Besonders bevorzugt ist dabei ein Verfahren, bei dem man in Schritt c) eine Hybridisierung der Amplifikate an zwei Klassen von Oligomeren (Oligonukleotiden und/oder PNA-Oligomeren) mit jeweils einem Mitglied für das besagte bestimmte Cytosin durchführt, wobei die Oligomere der ersten Klasse bevorzugt an die Sequenz hybridisieren, welche aus der chemischen Behandlung der genomischen DNA hervorgeht, wenn das besagte bestimmte Cytosin in der genomischen DNA methyliert vorläge und wobei die Oligomere der zweiten Klasse bevorzugt an die Sequenz hybridisieren, welche aus der chemischen Behandlung der genomischen DNA hervorgeht, wenn das besagte bestimmte Cytosin in der genomischen DNA unmethyliert vorläge. Eine dieser zwei Klassen von Oligmeren koennen beispielsweise durch Oligonukleotide, die ein CG in der Mitte enthalten gebildet werden und die andere Klasse durch Oligonukeotide, welche in der Mitte ein TG (oder ein CA, im Gegenstrang) aufweisen. Die restlichen Teile der Oligomersequenzen sollten zwischen beiden Klassen bevorzugt übereinstimmen. In diesem Fall würden Oligonukleotide der ersten Klasse an die Sequenz (um das-zu untersuchende bestimmte Cytosin) hybridisieren, wie sie nach Bisulfit Umsetzung in methylierten Fall vorläge, umgekehrt würden die der zweiten Klasse an die Sequenz hybridisieren, wie sie nach Bisulfit Umsetzung in unmethylierten Fall vorläge.

Besonders bevorzugt ist auch ein Verfahren, bei dem man in Schritt c) eine Hybridisierung der Amplifikate an zwei Klassen von Oligomeren (Oligonukleotiden und/oder PNA-Oligomeren) mit jeweils mindestens einem Mitglied durchführt, wobei die Oligomere der ersten Klasse bevorzugt an die Sequenz hybridisieren, welche aus der chemischen Behandlung der genomischen DNA hervorgeht, wenn das besagte bestimmte Cytosin in der genomischen DNA methyliert vorläge und weniger bevorzugt an die Sequenz, welche aus der chemischen Behandlung der genomischen DNA hervorgeht, wenn das besagte bestimmte Cytosin in der genomischen DNA unmethyliert vorläge und wobei die Oligomere der zweiten Klasse an das zu untersuchende Amplifikat im wesentlichen unabhängig von dem Methylierungsgrad besagten bestimmten Cytosins in der genomischen DNA hybridisieren.

Ensprechend ist auch ein Verfahren bevorzugt, bei dem man in Schritt c) eine Hybridisierung der Amplifikate an zwei Klassen von Oligomeren (Oligonukleotiden und/oder PNA-Oligomeren) mit jeweils einem Mitglied für das besagte bestimmte Cytosin erfolgt, wobei die Oligomere der ersten Klasse bevorzugt an die Sequenz hybridisieren, welche aus der chemischen Behandlung der genomischen DNA hervorgeht, wenn das besagte bestimmte Cytosin in der genomischen DNA unmethyliert vorläge und weniger bevorzugt an die Sequenz, welche aus der chemischen Behandlung der genomischen DNA hervorgeht, wenn das besagte bestimmte Cytosin in der genomischen DNA methyliert vorläge und wobei die Oligomere der zweiten Klasse an das zu untersuchende Amplifikat im wesentlichen unabhängig von dem Methylierungsgrad besagten bestimmten Cytosins in der genomischen DNA hybridisieren.

In diesen Fällen hybridisiert also die zweite Klasse von Oligomeren an das Amplifikat, ohne dass sich eine wesentliche Methylierungsspezifität ergibt, demnach also bevorzugt an eine Position des Amplifikates die keinen methylierbaren Cytosin Positionen entspricht. Damit wird letztlich nur die Konzentration des Amplifikates über die Intensität der Hybridisierung bestimmt. Relativ dazu erfolgt die Hybridisierung an die erste Klasse von Oligonukleotiden in Abhängigkeit von dem Methylierungsgrad der zu untersuchenden bestimmten Cytosins.

Bevorzugt ist es, dass man das Verfahren nicht nur mit der genomischen DNA-Probe durchführt, sondern sinngemäß auch mit bekanntermaßen an der Position des besagten bestimmten Cytosins methyliert oder aber unmethyliert vorliegender Standard-DNA, wobei die jeweils mit der unmethylierten Standard DNA gemessenen Verhältnisse der an den beiden Klassen von Oligonukleotiden detektierten Markierungen als Eichwert für einen Methylierungsgrad von 0 und entsprechend die jeweils mit der methylierten Standard DNA gemäss gemessenen Verhältnisse der an den beiden Klassen von Oligonukleotiden detektierten Markierungen als Eichwert für einen Methylierungsgrad von 1 dienen und man diese Eichwerte für die Bestimmung des Methylierungsgrades der genomischen Proben-DNA einsetzt.

Besonders bevorzugt ist es, dass man weitere bekannte Standard DNA-Proben zur Eichung verwendet, die jeweils einen beliebigen bekannten Methylierungsgrad des besagten bestimmten Cytosins aufweisen.

Die verwendeten Standard DNA-Proben und die Probe (das aus einer genomischen DNA hergestellte Amplifikat) sind bevorzugt jeweils unterschiedlich markiert. Die verwendeten Standard DNA Proben sind jewiels wiederum bevorzugt unterschiedlich markiert.

Besonders bevorzugt ist auch ein Verfahren, bei dem man Amplifikate, hervorgegangen aus verschiedenen genomischen DNA-Proben, mit unterschiedlichen Markierungen versieht. In diesem Fall ist es möglich, mit einem Satz von Oligonukleotiden der beiden Klassen gleichzeitig verschiedene Proben zu messen, sei es beipielsweise auf einem Oligomer Array, welcher Oligonukleotide der beiden Klassen enthält.

Beosnders bevorzugt ist auch ein Verfahren, bei dem man Amplifikate, hervorgegangen aus derselben genomischen DNA-Proben, mit unterschiedlichen Markierungen versieht, um über eine Mittelung der aus verschiedenen Detektionsverfahren erhaltenen Werte eine Erhöhung der Messgenauigkeit zu erreichen. Dies kann beipielsweise durch Markierungen mit unterschiedlichen Fluoreszenzfarbstoffen erfolgen. Die Messung erfolgt in diesem Fall mittels eines Fluoreszenzscanners, welcher über mehrere Kanäle zur Messung der individuellen Emissionswellenlängen der Fluoreszenzfarbstoffe verfügt.

Besonders bevorzugt ist demnach auch ein Verfahren, bei dem die Markierungen Fluoreszenzmarkierungen sind.

Bevorzugt ist es erfindungsgemäß ferner, dass besagte Markierung eine Fluoreszenzmarkierung ist. Dabei ist bevorzugt, dass man besagte Markierung durch Chemilumineszenz, ihre UV-Absorption oder Fluoreszenzpolarisation nachweist.

Ganz besonders ist es erfindungsgemäß bevorzugt, dass man die Behandlung der DNA in Schritt a) mit einer Lösung eines Bisulfits (=Hydrogensulfit, Disulfit) durchführt.

Bevorzugt ist auch, dass die Markierungen Radionuklide sind.

Bevorzugt ist ferner, dass die Markierungen ablösbare Massenmarkierungen sind, die in einem Massenspektrometer nachgewiesen werden.

Erfindungsgemäß besonders bevorzugt ist es, dass man die PCR-Produkte insgesamt oder deren charakteristische Fragmente im Massenspektrometer nachweist und somit durch ihre Masse eindeutig charakterisiert sind.

Erfindungsgemäß besonders bevorzugt ist es, dass die Oligomere (Oligonukleotide und/oder PNA-Oligomere) einer Klasse die Sequenz 5'-CG-3' umfassen.

Erfindungsgemäß besonders bevorzugt ist es, dass die Oligomere (Oligonukleotide und/oder PNA-Oligomere) einer Klasse die Sequenz 5'-TG-3' und/oder die Sequenz 5'-CA-3' umfassen.

Besonders bevorzugt ist es ferner auch, dass die Oligonukleotide der ersten Klasse die Sequenz 5'-CG-3' umfassen und die Oligonukleotide der zweiten Klassen die Sequenz 5'-TG-3' und/oder die Sequenz 5'-CA-3' umfassen.

Bevorzugt ist es auch, dass die Oligonukleotide der ersten und der zweiten Klasse auf einer gemeinsamen Festphase immobilisiert sind. Auch ist dabei bevorzugt, dass die Oligonukleotide auf einer ebenen Festphase in einem rechtwinkligen oder hexagonalen Raster angeordnet sind und der Ort bestimmter Oligonukleotide auf der Festphase mit deren jeweiliger Sequenz korreliert ist.

Ebenfalls besonders bevorzugt ist es, dass die Oligomere der ersten und zweiten Klasse auf Beads immobilisiert sind, welche mit einem Satz getrennt nachweisbarer Markierungen codiert sind. Letztere dienen zur Identifizierung des Beads, d.h. der an das betreffende Bead gebundenen Sequenzen. Die an die Beads gebundenen Amplifikate werden dann mittels anderer Markierungen, welche an die Amplifikate gebunden sind, identifiziert. Instrumente zur Durchführung solcher Messungen basierend auf Beads werden beipsielsweise von der Firma Luminex angeboten.

Ganz besonders bevorzugt ist ein erfindungsgemäßes Verfahren, wobei man den Schritt b) in zwei Teilschritten wie folgt durchführt:
a) eine PCR Präamplifikation mit mindestens einem Primerpaar unterschiedlicher Sequenz, die an eine nach Anspruch 1 vorbehandelte DNA-Probe unspezifisch hybridisieren und daher im PCR Schritt mehr als ein Amplifikat ergeben;
b) eine PCR Amplifikation des in der Präamplifikation gebildeten Produkts mit Primern unterschiedlicher Sequenz, die jeweils zu einem Abschnitt der nach Anspruch 1 vorbehandelten DNA-Probe [(+)-Strang oder (-)-Strang] identisch oder revers komplementär sind und an die zu amplifizierende DNA spezifisch hybridisieren.

Unter Hybridisierung wird im Zusammenhang dieser Erfindung eine Hybridisierung zweier gemäß den Watson-Crick Regeln vollständig zueinander revers komplementärer DNA-Einzelstränge verstanden, ohne dass eine Basenfehlpaarung auftritt. Uracil wird in diesem Zusammenhang wie Thymin betrachtet.

Erfindungsgemäß bevorzugt ist es ferner, dass die Amplifikation von mehreren DNA-Abschnitten in einem Reaktionsgefäß durchführt wird.

Weiterhin ist erfindungsgemäß bevorzugt, dass für die Amplifikation eine hitzebeständige DNA-Polymerase verwendet wird. Besonders bevorzugt ist auch, dass die für die Amplifikation verwendeten Primeroligonukleotide der entweder nur die Basen T, A und C oder aber die Basen T, A und G enthalten.

Bevorzugt ist es auch, dass mindestens 10 CpG Positionen in unterschiedlichem Sequenzkontext gleichzeitig analysiert werden. Besonders bevorzugt ist es, dass mindestens 50 CpG Positionen in unterschiedlichem Sequenzkontext gleichzeitig analysiert werden. Ganz besonders bevorzugt ist es, dass mindestens 100 CpG Positionen in unterschiedlichem Sequenzkontext gleichzeitig analysiert werden. Höchst bevorzugt ist es, dass mindestens 500 CpG Positionen in unterschiedlichem Sequenzkontext gleichzeitig analysiert werden. Am bevorzugsten ist es, dass mindestens 1000 CpG Positionen in unterschiedlichem Sequenzkontext gleichzeitig analysiert werden.

Erfindungsgemäß bevorzugt ist das Verfahren, wobei die genomische DNA-Probe aus Zelllinien, Blut, Sputum, Stuhl, Urin, Gehirn-Rückenmarks-Flüssigkeit, in Paraffin einbettetem Gewebe, beispielsweise Gewebe von Augen, Darm, Niere, Hirn, Herz, Prostata, Lunge, Brust oder Leber, histologischen Objektträgern oder allen möglichen Kombinationen hiervon erhalten wurde.

Bevorzugt ist die Verwendung eines erfindungsgemäßen Verfahrens zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische und psychologische Konsequenzen von Gehirnschädigungen ; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

Weiterhin ist die Verwendung eines erfindungsgemäßen Verfahrens zur Unterscheidung von Zelltypen oder Geweben oder zur Untersuchung der Zelldifferenzierung bevorzugt.

Gegenstand der vorliegenden Erfindung ist ein Kit, umfassend ein Bisulfit enthaltendes Reagenz, Primeroligonukleotiden zur Herstellung der Amplifikate und/oder vorzugsweise an einer Festphase immobilisierte Oligonukleotiden sowie eine Anleitung zur Durchführung des erfindungsgemäßen Verfahrens.

Diese genomische DNA-Probe wird bevorzugt aus Zelllinien, Blut, Sputum, Stuhl, Urin, Gehirn-Rückenmarks-Flüssigkeit, in Paraffin einbettetem Gewebe, beispielsweise Gewebe von Augen, Darm, Niere, Hirn, Herz, Prostata, Lunge, Brust oder Leber, histologischen Objektträgern oder allen möglichen Kombinationen hiervon erhalten.

Bei diesem Verfahren wird im ersten Schritt eine genomische DNA-Probe derart behandelt, dass außer den 5-Methylcytosinbasen alle Cytosinbasen in Uracil umgewandelt werden. Diese chemische Behandlung wird bevorzugt mit der Lösung eines Bisulfits (=Hydrogensulfit, Disutfit) durchgeführt.

Dieser Verfahrensschritt kann nicht nur mit der genomischen DNA-Probe durchgeführt werden, sondern vorzugsweise sinngemäß auch mit bekanntermaßen an der Position des besagten bestimmten Cytosins methyliert oder aber unmethyliert vorliegender Standard-DNA.

Im zweiten Schritt des Verfahrens werden die Teile der genomischen DNA amplifiziert, die das besagte bestimmte Cytosin enthalten. Dieser Schritt kann besonders bevorzugt in zwei Teilschritten durchgeführt werden:
1. Zuerst wird eine PCR Präamplifikation mit mindestens einem Primerpaar unterschiedlicher Sequenz durchgeführt, die an eine chemisch vorbehandelte DNA hybridisieren. Diese Vorbehandlung erfolgte chemisch derart, dass die Cytosinbasen in Uracil umgewandelt wurden, nicht aber die 5-Methylcytosinbasen.
2. Eine PCR-Amplifikation des in der Präamplifikation gebildeten Produkts wird mit Primern unterschiedlicher Sequenz durchgeführt. Diese Primer sind identisch oder revers komplementär zu einem Abschnitt des chemisch vorbehandelten DNA [(+)-Strangs oder (-)-Strangs] und hybridisieren spezifisch an die zu amplifizierende DNA. Die Amplifikate enthalten vorzugsweise eine nachweisbare Markierung.

Im folgenden dritten Schritt des Verfahrens erfolgt eine Hybridisierung der Amplifikate an bevorzugt zwei Klassen von Oligonukleotiden mit jeweils einem Mitglied. In einer besonders bevorzugten Variante des Verfahrens umfassen die Oligonukleotide der ersten Klasse die Sequenz 5'-CG-3' und die Oligonukleotide der zweiten Klasse die Sequenz 5'-TG-3' und/oder die Sequenz 5'-CA-3'. Die Oligonukleotide der ersten und der zweiten Klasse sind vorzugsweise auf einer gemeinsamen Festphase immobilisiert. Die Oligonukleotide sind auf einer ebenen Festphase in einem rechtwinkligen oder hexagonalen Raster angeordnet und der Ort der bestimmten Oligonukleotide ist auf der Festphase mit der jeweiligen Sequenz korreliert.

Die Oligonukleotide der ersten Klasse hybridisieren bevorzugt an die Sequenz, welche aus der chemischen Behandlung der genomischen DNA hervorgeht, wenn das besagte bestimmte Cytosin in der genomischen DNA methyliert vorläge. Die Oligonukleotide der zweiten Klasse hybridisieren bevorzugt an die Sequenz, welche aus der chemischen Behandlung der genomischen DNA hervorgeht, wenn das besagte bestimmte Cytosin in der genomischen DNA unmethyliert vorläge.

Die Amplifikation von mehreren DNA-Abschnitten wird besonders bevorzugt in einem Reaktionsgefäß durchgeführt. Bevorzugt führt man die Amplifikation mit der Polymerasekettenreaktion (PCR) durch, wobei vorzugsweise eine hitzebeständige DNA-Polymerase verwendet wird.

Die für die Amplifikation verwendeten Primeroligonukleotide enthalten bevorzugt entweder nur die Basen T, A und C oder aber die Basen T, A und G.

Im vierten Verfahrensschritt wird das Ausmaß der Hybridisierung der Amplifikate an die zwei Klassen von Oligonukleotiden durch die Detektion der Markierungen der Amplifikate bestimmt. Die Markierungen sind besonders bevorzugt Fluoreszenzmarkierungen, Radionuklide oder ablösbare Massenmarkierungen, die in einem Massenspektrometer nachgewiesen werden. Die Markierung werden vorzugsweise ebenfalls durch Chemilumineszenz, UV-Absorption oder Fluoreszenzpolarisation nachgewiesen. Die PCR-Produkte können auch bevorzugt insgesamt oder als deren charakteristische Fragmente im Massenspektrometer nachgewiesen werden. Somit sind die PCR-Produkte durch ihre Masse eindeutig charakterisiert.

Im letzten Verfahrensschritt wird aus dem Verhältnis der an den zwei Klassen von Oligonukleotiden in Folge der Hybridisierung detektierten Markierungen auf den Methylierungsgrad des besagten bestimmten Cytosins in der genomischen DNA-Probe geschlossen.

Die jeweils mit der unmethylierten Standard-DNA gemessenen Verhältnisse der an den beiden Klassen von Oligonukleotiden detektierten Markierungen dienen vorzugsweise als Eichwert für einen Methylierungsgrad von 0.

Entsprechend dienen die jeweils mit der methylierten Standard-DNA gemessenen Verhältnisse der an den beiden Klassen von Oligonukleotiden detektierten Markierungen vorzugsweise als Eichwert für einen Methylierungsgrad von 1. Diese Eichwerte werden besonders bevorzugt für die Bestimmung des Methylierungsgrades der genomischen DNA-Proben eingesetzt.

Für die Eichung können vorzugsweise auch weitere bekannte Standard DNA-Proben zur Eichung verwendet werden, die jeweils einen beliebigen bekannten Methylierungsgrad des besagten bestimmten Cytosins aufweisen.

Das Verfahren ist dadurch charakterisiert, dass bevorzugt mindestens 10 CpG Positionen in unterschiedlichem Sequenzkontext gleichzeitig analysiert werden. Ferner können vorzugsweise mindestens 50 CpG Positionen in unterschiedlichem Sequenzkontext gleichzeitig analysiert werden. Bevorzugt ist außerdem, dass mindestens 100 CpG Positionen in unterschiedlichem Sequenzkontext gleichzeitig analysiert werden. Sehr bevorzugt ist die gleichzeitige Analyse von mindestens 500 CpG Positionen in unterschiedlichem Sequenzkontext. Besonders bevorzugt ist abschließend die gleichzeitige Analyse von mindestens 1000 CpG Positionen in unterschiedlichem Sequenzkontext.

Das beschriebene Verfahren wird bevorzugt verwendet zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

Das vorliegende Verfahren wird besonders bevorzugt verwendet zur Unterscheidung von Zelltypen oder Geweben oder zur Untersuchung der Zelldifferenzierung.

Durch Bestimmung der Hybrisierungsverhältnisse zwischen den zwei eingesetzten Klassen von Oligonukleotiden (z.B. enthaltend CG/TG) wird Unabhängigkeit von der Intensität der Gesamthybridisierung der unbekannten Gewebeproben erreicht.

Zur Kalibrierung von unbekannten Gewebeproben werden unmethylierte und aufmethylierte Vergleichsproben als Standards eingesetzt.

Bestandteil dieses Verfahrens ist ferner ein Kit, das ein Bisulfit enthaltendes Reagenz, Primeroligonukleotide zur Herstellung der Amplifikate und/oder vorzugsweise an eine Festphase immobilisierte Oligonukleotide umfasst. Die Oligonukleotide (erste Klasse) umfassen die Sequenz 5'-CG-3'. Die Oligonkleotide (zweite Klasse) umfassen die Sequenz 5'-TG-3' und/oder die Sequenz 5'-CA-3'. Zu dem Kit gehört auch eine Anleitung zur Durchführung des Verfahrens.

Die Oligomere können mindestens ein CpG Dinukleotid enthalten. Das Cytosin des entsprechenden CpG Dinukleotids befindet sich in etwa im mittleren Drittel des Oligomers.

Die Oligomere können auf einem Trägermaterial in einer fixierten Anordnung hergestellt werden, wobei mindestens ein Oligomer an eine feste Phase gekoppelt wird. Verfahren zur Bindung von Oligomersonden an Festphasen sind dem Fachmann bekannt.

Möglich ist es, dass alle Oligomersonden die gleiche Länge haben. Besonders bevorzugt sind weiterhin alle 18mere, welche ein CG Dinukleotid in der Mitte aufweisen und die an eine der Seq. ID 1 bis Seq. ID 40712 ohne Basenfehlpaarungen hybridisieren.

Die Oligomere können verwendet werden zur Diagnose von unerwünschten Arzneimittelwirkungen, Krebserkrankungen, CNS-Fehlfunktionen, Schäden oder Erkrankungen, aggressiven Symptomen oder Verhaltensstörungen, klinischen und psychologischen Folgen von Gehirnverletzungen, psychotische Störungen und Persönlichkeitsstörungen, Demenz und/oder assoziierten Syndromen, kardiovaskulärer Krankheit, Fehlfunktion, Schädigung oder Erkrankung des gastrointestinalen Traktes, Fehlfunktion, Schädigung oder Erkrankung des Atmungssystems, Verletzung, Entzündung, Infektion, Immunität und/oder Rekonvaleszenz, Fehlfunktion, Schädigung oder Erkrankung des Körpers als Abweichung im Entwicklungsprozess, Fehlfunktion, Schädigung oder Erkrankung der Haut, der Muskeln, des Bindegewebes oder der Knochen, endokrine und metabolische Fehlfunktion, Schädigung oder Erkrankung, Kopfschmerzen und sexuellen Fehlfunktionen durch Analyse von Methylierungsmustern.

Dem Fachmann wird verständlich sein, dass die Oligomere bei einem Austausch von Thymin gegen Uracil in den verwendeten Sequenzen den gleichen Zweck erfüllen.

Die zu analysierende genomische DNA wird bevorzugt aus den üblichen Quellen für DNA erhalten, wie z. B. Zelllinien, Blut, Sputum, Stuhl, Urin, Gehirn-Rückenmarks-Flüssigkeit, in Paraffin eingebettetes Gewebe, beispielsweise Gewebe von Augen, Darm, Niere, Hirn, Herz, Prostata, Lunge, Brust oder Leber, histologische Objektträger und alle möglichen Kombinationen hiervon.

Die folgenden Beispiele erläutern die Erfindung.

### Beispiel 1:

Herstellung von nicht- und aufmethylierter DNA und Bisulphit-Behandlung

Für die Herstellung aufmethylierter DNA wurden humane genomische DNA mit S-Adenosylmethion und der CpG Methylase (SssI, New England Biolabs,) nach Angaben des Herstellers behandelt. Für die Herstellung nichtmethylierter DNA wurde das Genfragment ELK-1 (Accession number ep59011) mit den Primer [SEQ ID No: 1] GCTCTATGGTCTTGTCTAACCGTA und [SEQ ID No: 2] AGGTGGTGGTGGCGGTGG ausgehend von humaner genomischer DNA, mittels PCR amplifiziert. Die so hergestellte nicht-und aufmethylierter DNA, wie auch humane genomische DNA, wurde unter Verwendung von Bisulphit (Hydrogensulfit, Disulfit) derart behandelt, dass alle nicht an der 5-Position der Base methylierten Cytosine so verändert werden, dass eine hinsichtlich dem Basenpaarungsverhalten unterschiedliche Base entsteht, während die in 5-Position methylierten Cytosine unverändert bleiben. Wird für die Reaktion Bisulfit im Konzentrationsbereich zwischen 0.1 M und 6 M verwendet, so findet an den nicht methylierten Cytosinbasen eine Addition statt. Zudem müssen ein denaturierendes Reagenz oder Lösungsmittel sowie ein Radikalfänger zugegen sein. Eine anschließende alkalische Hydrolyse führt dann zur Umwandlung von nicht methylierten Cytosin-Nukleobasen in Uracil. Diese umgewandelte DNA dient dazu, methylierte Cytosine nachzuweisen.

### Beispiel 2:

### Herstellung der Cy5-markierten Gensonden

Ausgehend von den Bisulphit behandelten DNA Proben wird je ein definiertes Fragment der Länge 529 bp aus der Promoterregion des ELK-1 Gens amplifiziert (Accession number ep59011). Die Amplifikation wird mit den Primeroligonukleotiden [SEQ ID No: 3] ATGGTTTTGTTTAATYGTAGAGTTGTTT und [SEQ ID No: 4] TAAACCCRAAAAAAAAAAACCCAATAT durchgeführt. Durch Verwenden von Primeroligonukleotiden, die mit dem Fluoreszenfarbstoff Cy5 markiert sind, wird das Fragment direkt bei der PCR markiert. Als Matrix DNA wird Bisulphit (Hydrogensulfit, Disulfit) behandelte (1) nichtmethylierte, (2) aufmethylierte und (3) humane genomische DNA verwendet. Anschließend werden diese drei unterschiedlichen DNA-Fragmente in getrennten Hybridisierungen auf ihren Methylierungsgrad an einer spezifischen CpG Position untersucht.

### Beispiel 3:

### Durchführung der Hybridisierung und Auswertung eines hybridisierten DNA-"Chip"

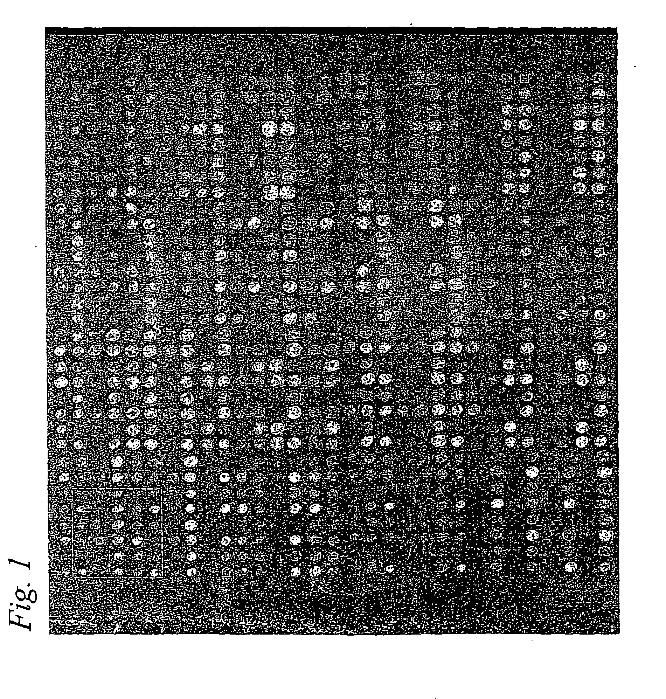

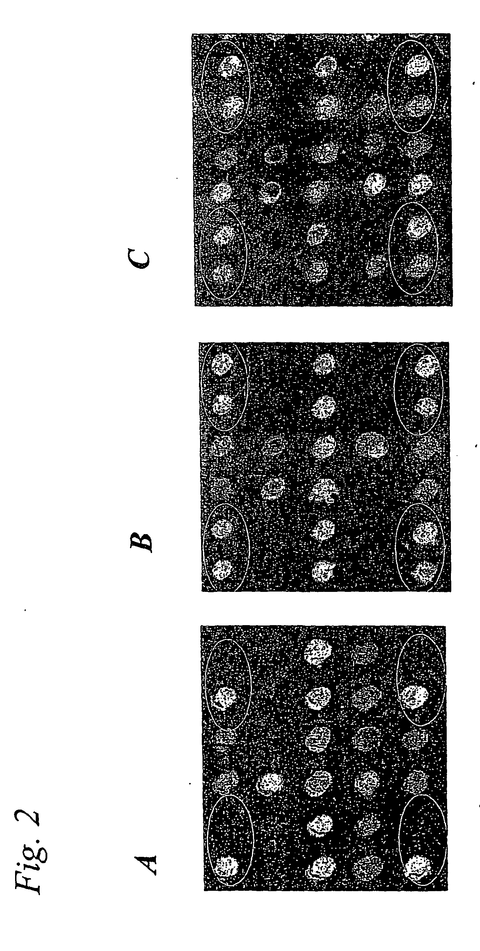

Die in Beispiel 2 hergestellte Gensonden wird auf einem DNA Chip hybridisiert. Auf dem Chip sind zuvor Oligonukleotide immobilisiert worden. Die Oligonukleotidesequenzen leiten sich von dem in Beispiel 2 genannten amplifizierten Fragment des Gens ELK-1 ab, und repräsentierten die CG Dinukleotide, die unmittelbare Umgebung einschließend. Die Länge der Oligonukleotide beträgt 14-22 Nukleotide, die Position des CG Dinukleotides innerhalb der Oligonukleotide ist variabel. Nach der Hybridisierung wird der DNA Chip gescannt (siehe Fig. 1) und die Hybridisierungssignale numerisch ausgewertet (Daten nicht gezeigt). Das Ergebnis der Hybridisierung für die Oligonukleotide [SEQ ID No: 5] CTACTCAACGAAAACAAA und [SEQ ID No: 6] CTACTCAACAAAAACAAA wird in Fig. 1 und Fig. 2 gezeigt. Hierbei hybridisiert [SEQ ID No: 5] CTACTCAACGAAAACAAA bevorzugt, wenn das zu Cytosin des ELK-1 Fragments, welches sich an Position 103 des Amplifikates befindet, methyliert ist, [SEQ ID No: 6] CTACTCAACAAAAACAAA wenn dieses Cytosin nicht-methyliert ist.

In Figur 1 ist ein DNA Chip nach Hybridisierung mit dem ELK-1 Fragment dargestellt. Dargestellt ist das Falschfarben-Bild wie es nach dem Scannen erzeugt wird. Entgegen der hier dargestellten schwarz-weiß Abbildung wird von dem Scanner ein farbiges Bild erzeugt. Die Intensität der verschiedenen Farben repräsentiert den Grad der Hybridisierung, wobei der Hybridisierungsgrad von rot (in Figur 1 als helle Spots zu erkennen) nach blau (in Figur 1 als dunkle Spots zu erkennen) abnimmt.

Figur 2 A stellt eine Teilabbildung von Figur 1 dar. Zur Verdeutlichung des Hybridisierungsschemas sind die gespotteten Oligonkleotidpaare weiß umrandet: [SEQ ID No: 6] ctactcaacaaaaacaaa (links) und [SEQ ID No: 5] ctactcaacgaaaacaaa (rechts).

Teilabbildung Figur 2 B zeigt das Spottingmuster für einen unbekannten Methylierungsstatus der Gewebeprobe und Teilabbildung Figur 2 C den Methylierungsstatus für die aufmethylierte Vergleichsprobe.

**Tabelle 1:**

| | ***Probe A*** ***(unmethyliert)*** | | ***Probe B*** ***(unbekannt)*** | | ***Probe C*** ***(aufmethyliert)*** | |
|---|---|---|---|---|---|---|
| ***Sequenz des Detektionsoligomers*** | Fluoreszenz (counts) | ***Mittelwert*** | Fluoreszen z (counts) | ***Mittelwert*** | Fluoreszen z (counts) | ***Mittelwert*** |
| ctactcaacgaaaacaaa [SEQ ID No: 5] | 3352 | | 6102 | | 6002 | |
| ctactcaacgaaaacaaa [SEQ ID No: 5] | 2950 | | 6775 | | 7898 | |
| ctactcaacgaaaacaaa [SEQ ID No: 5] | 4196 | | 6360 | | 7485 | |
| ctactcaacgaaaacaaa [SEQ ID No: 5] | 5181 | | 5521 | | 11401 | |
| | | 3920 | | 6190 | | 8197 |
| ctactcaacaaaaacaaa [SEQ ID No: 6] | 20577 | | 7074 | | 7290 | |
| ctactcaacaaaaacaaa [SEQ ID No: 6] | 19709 | | 9171 | | 9985 | |
| ctactcaacaaaaacaaa [SEQ ID No: 6] | 24130 | | 7603 | | 9286 | |
| ctactcaacaaaaacaaa [SEQ ID No: 6] | 21601 | | 9434 | | 12435 | |
| | | 21504 | | 8321 | | 9749 |
| CG/CA | | 0,28 | | 0,74 | | 0,84 |

Der Mittelwert ist jeweils für eine Wellenlänge von 635 nm angegeben. In Spalte A werden die Werte für die unmethylierte Probe, in Spalte B für einen unbekannten Methylierungsstatus einer Gewebeprobe und in Spalte C die Werte für die aufmethylierte Vergleichsprobe. Die CG/CA Verhältnisse repräsentieren den Methylierungsstatus der jeweiligen Probe. Der Wert von 0,74 zeigt, dass die Probe im wesentlichen in aufmethylierter Form vorliegt.

### Beispiel 4 :

Das folgende Beispiel bezieht sich auf ein Fragment des mit dem vererbbaren non-polyposis Colorektalkrebs assoziierten Gens hMLH1, in dem eine bestimmte CG-Position auf Methylierung untersucht wird.

Im ersten Schritt wird eine genomische Sequenz unter Verwendung von Bisulfit (Hydrogensulfit, Disulfit) derart behandelt, dass alle nicht an der 5-Position der Base methylierten Cytosine so verändert werden, dass eine hinsichtlich dem Basenpaarungsverhalten unterschiedliche Base entsteht, während die in 5-Position methylierten Cytosine unverändert bleiben. Wird für die Reaktion Bisulfit im Konzentrationsbereich zwischen 0.1 M und 6 M verwendet, so findet an den nicht methylierten Cytosinbasen eine Addition statt. Zudem müssen ein denaturierendes Reagenz oder Lösungsmittel sowie ein Radikalfänger zugegen sein. Eine anschließende alkalische Hydrolyse führt dann zur Umwandlung von nicht methylierten Cytosin-Nukleobasen in Uracil. Diese umgewandelte DNA dient dazu, methylierte Cytosine nachzuweisen. Im zweiten Verfahrensschritt verdünnt man die behandelte DNA-Probe mit Wasser oder einer wässrigen Lösung. Bevorzugt wird anschliessend eine Desulfonierung der DNA (10-30 min, 90-100 °C) bei alkalischem pH-Wert durchgeführt. Im dritten Schritt des Verfahrens amplifiziert man die DNA-Probe in einer Polymerasekettenreaktion, bevorzugt mit einer hitzebeständigen DNA-Polymerase. Im vorliegenden Beispiel werden Cytosine des Gens hMLH1, hier aus einer 1551 bp großen 5' flankierenden Region, untersucht. Dazu wird mit den spezifischen Primeroligonukleotiden [SEQ ID No: 7] AGCAACACCTCCATGCACTG und [SEQ ID No: 8] TTGATTGGACAGCTTGAATGC ein definiertes Fragment der Länge 719 bp amplifiziert. Dieses Amplifikat dient als Probe, die an ein vorher an einer Festphase gebundenes Oligonukleotid unter Ausbildung einer Duplexstruktur hybridisiert, beispielsweise [SEQ ID No: 3] GAAGAGCGGACAG, wobei sich das nachzuweisende Cytosin an Position 588 des Amplifikats befindet. Der Nachweis des Hybridisierungsprodukts beruht auf Cy3 und Cy5 fluoreszenzmarkierten Primeroligonukleotiden, die für die Amplifikation verwendet wurden. Nur wenn in der Bisulfit behandelten DNA an dieser Stelle ein methyliertes Cytosin vorgelegen hat, kommt es zu einer Hybridisierungsreaktion der amplifizierten DNA mit dem Oligonukleotid. Somit entscheidet der Methylierungsstatus des jeweiligen zu untersuchenden Cytosins über das Hybridisierungsprodukt.

### Beispiel 5:

### Herstellung von durch Bisulfit modifizierter DNA durch Agarose beads

In dem vorliegenden Experiment wird, ausgehend von mit Bisulfit behandelter DNA, ein definiertes Fragment der Länge 529 bp aus der Promoterregion des ELK-1 Gens (Accession number ep59011) amplifiziert. Durch Verwenden der Primeroligonukleotide [SEQ ID No: 3] ATGGTTTTGTTTAATYGTAGAGTTGTTT und [SEQ ID No: 4] TAAACCCRAAAAAAAAAAACCCAATAT, die mit dem Fluoreszenfarbstoff ALEXA 488 markiert sind, wird das Fragment direkt bei der PCR markiert. Oligonukleotide der ersten Klasse (hier beispielsweise [SEQ ID No: 10] ATTAATAGCGTTTTGGTT) und der zweiten Klasse (hier: beispielsweise [SEQ ID No: 11] ATTAATAGTGTTTTGGTT) werden an den Oberflächen von beads immobilisiert, die sich durch eine individuelle Farbkodierung unterscheiden. In einem folgenden Schritt werden die mit den oben genannten Primeroligonukleotiden hergestellten Amplifikate des Gens ELK-1, mit einem Gemisch beider Klassen der beads zusammengebracht, wobei die Amplifikate gegen die immobilisierten Oligonukleotide, hier beispielsweise [SEQ ID No: 10] ATTAATAGCGTTTTGGTT und [SEQ ID No: 11] ATTAATAGTGTTTTGGTT, wobei sich das nachzuweisende C bzw. T jeweils an Position 476 des Amplifikats befindet, hybridisieren. Anschließend werden die beads vereinzelt, dann durch Fluoreszenzmessung anhand ihrer Farbkodierung identifiziert und durch Messung der Fluoreszenintensitäten, die spezifisch für den Fluoreszenzfarbstoff ALEXA 488 sind, der Grad der Hybridisierung ermittelt.

### Beispiel 6:

### Verwendung von multiplen Farbstoffen zur internen Kalibrierung

Ausgehend von mit Bisulfit behandelter DNA wird ein definiertes Fragment der Länge 529 bp aus der Promoterregion des ELK-1 Gens amplifiziert. Durch Verwenden fluoreszenzmarkierter Primeroligonukleotide [SEQ ID No: 3] ATGGTTTTGTTTAATYGTAGAGTTGTTT und [SEQ ID No: 4] TAAACCCRAAAAAAAAAAACCCAATAT, wird das Fragment direkt bei der PCR markiert. Für die PCR Reaktion, die auf einem Thermocycler (Eppendorf GmbH) durchgeführt wird, werden 10 ng von mit Bisulfit behandelter DNA, 6 pmol je Primer, 200 µM je dNTP, 1.5 mM MgCl₂ und 1 U HotstartTaq (Qiagen AG) eingesetzt. Die übrigen Bedingungen werden den Herstellerangaben gemäß gewählt. Für die Amplifikation wird eine Denaturierung für 14 min bei 96°C durchgeführt, woran sich 39 Zyklen mit den Bedingungen 60 sec bei 96°C, 45 sec bei 55 °C und 75 sec bei 72 °C anschließen. Zum Schluss wird eine Elongation für 10min bei 72°C durchgeführt. Im vorliegenden Fall werden die zu untersuchenden Proben, hier Gewebe von gesunden und kranken Personen, mit dem Farbstoff Cy2 markiert. Zur internen Kalibrierung des Methylierungsstatus werden Primeroligonukleotide zur Amplifikation der Proben für die Kalibrierung verwendet, die mit den Fluoreszenfarbstoffen Cy3 und Cy5 markiert sind. Die Amplifikate aus den Proben für die Kalibrierung repräsentieren einen bekannten Methylierungsstatus, zum einen, einen Zustand hundertprozentiger Methylierung und zum anderen einen nicht-methylierten Zustand. Da bei diesem Verfahren die Verhältnisse der Farbintensitäten der Fluoreszenzfarbstoffe, die unter Verwendung des ScanArray 4000XL, Packard BioScience-BioChip Technologies, gemessen werden, zwischen zwei Klassen von Oligonukleotiden, hier beispielsweise [SEQ ID No: 10] ATTAATAGCGTTTTGGTT und [SEQ ID No: 11] TTAATAGTGTTTTGGTT, wobei sich das nachzuweisende C bzw. T jeweils an Position 476 des Amplifikats befindet, berechnet werden, kann, das Verhältnis von methyliertem zu nicht methyliertem Zustand der unbekannten Proben ermittelt werden.

### Beispiel 7:

### Verwendung von multiplen Farbstoffen zur Erhöhung des Probendurchsatzvolumens und zur Erhöhung der Analysekomplexität

Das vorliegende Experiment dient dazu, verschiedene Proben in einem einzigen Hybridisierungsschritt zu analysieren und dadurch das Probendurchsatzvolumen zu erhöhen. Dazu wird von vier Individuen jeweils aus der Promotorregion des Gens ELK-1 jeweils ein definiertes Fragment der Länge 529 bp ausgehend von mit Bisulfit behandelter DNA, mit den Primeroligonukleotiden [SEQ ID No: 3] ATGGTTTTGTTTAATYGTAGAGTTGTTT und SEQ ID No: 4] TAAACCCRAAAAAAAAAACCCAATAT, amplifiziert. Diese von den vier Individuen stammenden Fragmente werden mit den vier verschiedenen Fluoreszenfarbstoffen Cy3, Cy5, Cy2 und Cy7 markiert und gegen immobilisierte Oligonukleotide, hier beispielsweise [SEQ ID No: 10] ATTAATAGCGTTTTGGTT und [SEQ ID No: 11] ATTAATAGTGTTTTGGTT, wobei sich das nachzuweisende C bzw. T jeweils an Position 476 des Amplifikats befindet, hybridisiert. Diese verschieden fluoreszenzmarkierten Proben werden anschließend bei unterschiedlichen Wellenlängen analysiert, ohne dass es zu einer gegenseitigen, auf einer Fluoreszenfarbstoff basierenden,Beeinflussung, kommt.
Umgekehrt ist es auch möglich, aus einer DNA Probe verschiedene unterschiedliche Sätze von Fragmenten zu erzeugen, die mit unterschiedlichen Farbstoffen markiert sind, hier Cy2, Cy3 und Cy5. Wird ein Satz von Oligonukleotidsonden für 64 Gene (Satz 1) auf einem Chip immobilisiert, so ist die Spezifität ausreichend, um 64 z.B. Cy3 markierte Fragmente unabhängig voneinander zu analysieren.. Dadurch, dass hier Proben von Satz 1 mit dem Fluoreszenzfarbstoff Cy3 und Proben von Satz 2 mit dem Fluoreszenzfarbstoff Cy5 markiert werden (und Satz 3 mit Cy2), wird die Detektion des Methylierungsstatus über die Fragmente des Satzes 1 bei der Messung der Fluoreszenzintensitäten nicht durch die fluoreszenzmarkierten Amplifikate der Sätze 2 und 3 beeinflusst (und umgekehrt). Damit ist es möglich, trotz erhöhter Komplexität der Amplifikate gleichermaßen zuverlässige Daten zu erzeugen wie bei einer Komplexität von 64 Amplifikaten.

### Beispiel 8:

### Verwendung von multiplen Farbstoffen zur Verifizierung der experimentellen Reproduzierbarkeit.

In dem vorliegenden Experiment werden gleiche PCR-Amplifikate mit vier verschiedenen Fluoreszenfarbstoffen markiert und diese in 4 facher Redundanz verifiziert. Dazu wird, ausgehend von mit Bisulfit behandelter DNA, ein definiertes Fragment der Länge 529 bp aus der Promoterregion des ELK-1 Gens mit den Primeroligonukleotiden [SEQ ID No: 3] ATGGTTTTGTTTAATYGTAGAGTTGTTT und [SEQ ID No: 4] TAAACCCRAAAAAAAAAAACCCAATAT, amplifiziert und gegen immobilisierte Oligonukleotide, hier beispielsweise [SEQ ID No: 10] ATTAATAGCGTTTTGGTT und [SEQ ID No: 11] ATTAATAGTGTTTTGGTT, wobei sich das nachzuweisende C bzw. T jeweils an Position 476 des Amplifikats befindet, hybridisiert. Durch Verwenden von mit Cy3, Cy5, Cy2 und Cy7 fluoreszenzmarkierten Primeroligonukleotiden werden die Verhältnisse der unterschiedlich fluoreszenzmarkierten Proben verglichen und auf diese Weise eine höhere experimentelle Sicherheit erzielt.

## Patentansprüche

1. Verfahren zur Detektion des Methylierungsgrades eines bestimmten Cytosins im Sequenzkontext 5'-CpG-3' einer genomischen DNA-Probe, **dadurch gekennzeichnet, dass**
a) man die genomische DNA chemisch behandelt, wobei man die Cytosinbasen in Uracil umwandelt, nicht aber die 5-Methylcytosinbasen;
b) man definierte Fragmente amplifiziert, die das besagte bestimmte Cytosin enthalten, wobei die Amplifikate eine nachweisbare Markierung erhalten;
c) man die Amplifikate an zwei Klassen von Oligonukleotiden und/oder PNA-Oligomeren mit jeweils einem Mitglied für das besagte bestimmte Cytosin hybridisiert ;
d) man das Ausmaß der Hybridisierung der Amplifikate an die zwei Klassen von Oligonukleotiden und/oder PNA-Oligomeren durch Detektion der Markierung der Amplifikate bestimmt;
e) man aus dem Verhältnis der an den zwei Klassen von Oligonukleotiden und/oder PNA-Oligomeren in Folge der Hybridisierung detektierten Markierungen auf den Methylierungsgrad besagten bestimmten Cytosins in der genomischen DNA-Probe schließt.

2. Verfahren nach Anspruch 1, **dadurch gekennzeichnet, dass** in Schritt c) eine Hybridisierung der Amplifikate an zwei Klassen von Oligomeren (Oligonukleotiden und/oder PNA-Oligomeren) mit jeweils einem Mitglied für das besagte bestimmte Cytosin erfolgt, wobei die Oligomere der ersten Klasse bevorzugt an die Sequenz hybridisieren, welche aus der chemischen Behandlung der genomischen DNA hervorgeht, wenn das besagte bestimmte Cytosin in der genomischen DNA methyliert vorläge und wobei die Oligomere der zweiten Klasse bevorzugt an die Sequenz hybridisieren, welche aus der chemischen Behandlung der genomischen DNA hervorgeht, wenn das besagte bestimmte Cytosin in der genomischen DNA unmethyliert vorläge.

3. Verfahren nach Anspruch 1, **dadurch gekennzeichnet, dass** in Schritt c) eine Hybridisierung der Amplifikate an zwei Klassen von Oligomeren (Oligonukleotiden und/oder PNA-Oligomeren) mit jeweils einem Mitglied für das besagte bestimmte Cytosin erfolgt, wobei die Oligomere der ersten Klasse bevorzugt an die Sequenz hybridisieren, welche aus der chemischen Behandlung der genomischen DNA hervorgeht, wenn das besagte bestimmte Cytosin in der genomischen DNA methyliert vorläge und weniger bevorzugt an die Sequenz, welche aus der chemischen Behandlung der genomischen DNA hervorgeht, wenn das besagte bestimmte Cytosin in der genomischen DNA unmethyliert vorläge und wobei die Oligomere der zweiten Klasse an das zu untersuchende Amplifikat im wesentlichen unabhängig von dem Methylierungsgrad besagten bestimmten Cytosins in der genomischen DNA hybridisieren.

4. Verfahren nach Anspruch 1, **dadurch gekennzeichnet, dass** in Schritt c) eine Hybridisierung der Amplifikate an zwei Klassen von Oligomeren (Oligonukleotiden und/oder PNA-Oligomeren) mit jeweils einem Mitglied für das besagte bestimmte Cytosin erfolgt, wobei die Oligomere der ersten Klasse bevorzugt an die Sequenz hybridisieren, welche aus der chemischen Behandlung der genomischen DNA hervorgeht, wenn das besagte bestimmte Cytosin in der genomischen DNA unmethyliert vorläge und weniger bevorzugt an die Sequenz, welche aus der chemischen Behandlung der genomischen DNA hervorgeht, wenn das besagte bestimmte Cytosin in der genomischen DNA methyliert vorläge und wobei die Oligomere der zweiten Klasse an das zu untersuchende Amplifikat im wesentlichen unabhängig von dem Methylierungsgrad besagten bestimmten Cytosins in der genomischen DNA hybridisieren.

5. Verfahren nach einem der Ansprüche 1 bis 4, **dadurch gekennzeichnet, dass** man das Verfahren nicht nur mit der genomischen DNA-Probe durchführt, sondern sinngemäß auch mit bekanntermaßen an der Position des besagten bestimmten Cytosins methyliert oder aber unmethyliert vorliegender Standard-DNA, wobei die jeweils mit der unmethylierten Standard DNA gemäss Anspruch 1 gemessenen Verhältnisse der an den beiden Klassen von Oligonukleotiden detektierten Markierungen als Eichwert für einen Methylierungsgrad von 0 und entsprechend die jeweils mit der methylierten Standard DNA gemäss Anspruch 1 gemessenen Verhältnisse der an den beiden Klassen von Oligonukleotiden detektierten Markierungen als Eichwert für einen Methylierungsgrad von 1 dienen und man diese Eichwerte für die Bestimmung des Methylierungsgrades der genomischen Proben-DNA einsetzt.

6. Verfahren nach Anspruch 5, **dadurch gekennzeichnet, dass** man weitere bekannte Standard DNA-Proben zur Eichung verwendet, die jeweils einen beliebigen bekannten Methylierungsgrad des besagten bestimmten Cytosins aufweisen.

7. Verfahren nach einem der Ansprüche 5 oder 6, **dadurch gekennzeichnet, dass** die als Standard verwendeten DNAs jeweils unterschiedlich markiert sind und die Probe (das aus einer genomischen DNA ensprechend Anspruch 1 hergestellte Amplifikat) wiederum mit einer davon verschiedenen Markierung versehen ist.

8. Verfahren nach einem der Ansprüche 1 bis 7, **dadurch gekennzeichnet, dass** Amplifikate, hervorgegangen aus verschiedenen genomischen DNA-Proben, mit unterschiedlichen Markierungen versehen sind.

9. Verfahren nach einem der Ansprüche 1 bis 8, **dadurch gekennzeichnet, dass** Amplifikate, hervorgegangen aus derselben genomischen DNA-Proben, mit unterschiedlichen Markierungen versehen sind, um über eine Mittelung der aus verschiedenen Detektionsverfahren erhaltenen Werte eine Erhöhung der Messgenauigkeit zu erreichen.

10. Verfahren nach einem der voranstehenden Ansprüche, **dadurch gekennzeichnet, dass** besagte Markierungen Fluoreszenzmarkierungen sind.

11. Verfahren nach einem der voranstehenden Ansprüche, **dadurch gekennzeichnet, dass** man die chemische Behandlung mit einer Lösung eines Bisulfits (=Hydrogensulfit, Disulfit) durchführt.

12. Verfahren nach einem der voranstehenden Ansprüche, **dadurch gekennzeichnet, dass** man besagte Markierung durch ihre Chemilumineszenz, ihre UV-Absorption oder Fluoreszenzpolarisation nachweist.

13. Verfahren nach einem der Ansprüche 1 bis 11, **dadurch gekennzeichnet, dass** die Markierungen Radionuklide sind.

14. Verfahren nach einem der Ansprüche 1 bis 11, **dadurch gekennzeichnet, dass** die Markierungen ablösbare Massenmarkierungen sind, die in einem Massenspektrometer nachgewiesen werden.

15. Verfahren nach einem der Ansprüche 1 bis 11, **dadurch gekennzeichnet, dass** man die PCR-Produkte insgesamt oder deren charakteristische Fragmente im Massenspektrometer nachweist und somit durch ihre Masse eindeutig charakterisiert sind.

16. Verfahren nach einem der voranstehenden Ansprüche, **dadurch gekennzeichnet, dass** die Oligomere (Oligonukleotide und/oder PNA-Oligomere) einer Klasse die Sequenz 5'-CG-3' umfassen.

17. Verfahren nach einem der voranstehenden Ansprüche, **dadurch gekennzeichnet, dass** die Oligomere (oligonukleotide und/oder PNA-Oligomere) einer Klasse die Sequenz 5'-TG-3' oder die Sequenz 5'-CA-3' umfassen.

18. Verfahren nach den Ansprüchen 16 und 17, **dadurch gekennzeichnet, dass** die Oligomere (Oligonukleotide und/oder PNA-Oligomere) der ersten Klasse die Sequenz 5'-CG-3' umfassen und die Oligomere der zweiten Klasse die Sequenz 5'-TG-3' oder die Sequenz 5'-CA-3' umfassen.

19. Verfahren nach einem der voranstehenden Ansprüche, **dadurch gekennzeichnet, dass** die Oligomere der ersten und der zweiten Klasse auf einer gemeinsamen Festphase immobilisiert sind.

20. Verfahren nach Anspruch 11, **dadurch gekennzeichnet, dass** die Oligonukleotide auf einer ebenen Festphase in einem rechtwinkligen oder hexagonalen Raster angeordnet sind und der Ort bestimmter Oligonukleotide auf der Festphase mit deren jeweiliger Sequenz korreliert ist.

21. Verfahren nach einem der Ansprüche 1-18, **dadurch gekennzeichnet, dass** die Oligomere der ersten und zweiten Klasse auf Beads immobilisiert sind, welche mit einem Satz getrennt nachweisbarer Markierungen codiert sind.

22. Verfahren nach einem der voranstehenden Ansprüche **dadurch gekennzeichnet, dass** man den Schritt b) des Anspruchs 1 in zwei Teilschritten wie folgt durchführt:
a) eine PCR Präamplifikation mit mindestens einem Primerpaar unterschiedlicher Sequenz, die an eine nach Anspruch 1 vorbehandelte DNA-Probe unspezifisch hybridisieren und daher im PCR Schritt mehr als ein Amplifikat ergeben;
b) eine PCR Amplifikation des in der Präamplifikation gebildeten Produkts mit Primern unterschiedlicher Sequenz, die jeweils zu einem Abschnitt der nach Anspruch 1 vorbehandelten DNA-Probe [(+)-Strang oder (-)-Strang] identisch oder revers komplementär sind und an die zu amplifizierende DNA spezifisch hybridisieren.

23. Verfahren nach einem der vorangehenden Ansprüche, **dadurch gekennzeichnet, dass** die Amplifikation von mehreren DNA-Abschnitten in einem Reaktionsgefäß durchführt wird.

24. Verfahren nach einem der voranstehenden Ansprüche, **dadurch gekennzeichnet, dass** für die Amplifikation eine hitzebeständige DNA-Polymerase verwendet wird.

25. Verfahren nach einem der voranstehenden Ansprüche, **dadurch gekennzeichnet, dass** die für die Amplifikation verwendeten Primeroligonukleotide der entweder nur die Basen T, A und C oder aber die Basen T, A und G enthalten.

26. Verfahren nach einem der voranstehenden Ansprüche, **dadurch gekennzeichnet, dass** mindestens 10 CpG Positionen in unterschiedlichem Sequenzkontext gleichzeitig analysiert werden.

27. Verfahren nach einem der voranstehenden Ansprüche, **dadurch gekennzeichnet, dass** mindestens 50 CpG Positionen in unterschiedlichem Sequenzkontext gleichzeitig analysiert werden.

28. Verfahren nach einem der voranstehenden Ansprüche, **dadurch gekennzeichnet, dass** mindestens 100 CpG Positionen in unterschiedlichem Sequenzkontext gleichzeitig analysiert werden.

29. Verfahren nach einem der voranstehenden Ansprüche, **dadurch gekennzeichnet, dass** mindestens 500 CpG Positionen in unterschiedlichem Sequenzkontext gleichzeitig analysiert werden.

30. Verfahren nach einem der voranstehenden Ansprüche, **dadurch gekennzeichnet, dass** mindestens 1000 CpG Positionen in unterschiedlichem Sequenzkontext gleichzeitig analysiert werden.

31. Verfahren nach einem der voranstehenden Ansprüche, wobei die genomische DNA-Probe aus zelllinien, Blut, Sputum, Stuhl, Urin, Gehirn-Rückenmarks-Flüssigkeit, in Paraffin einbettetem Gewebe, beispielsweise Gewebe von Augen, Darm, Niere, Hirn, Herz, Prostata, Lunge, Brust oder Leber, histologischen Objektträgern oder allen möglichen Kombinationen hiervon erhalten wurde.

32. Verwendung eines Verfahrens nach einem der voranstehenden Ansprüche zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen ; klinische, psychologische Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

33. Verwendung eines Verfahrens nach einem der voranstehenden Ansprüche zur Unterscheidung von Zelltypen oder Geweben oder zur Untersuchung der Zelldifferenzierung.

34. Kit, zur Durchführung eines Verfahrens nach einem der oben genannten Ansprüche umfassend
(a) ein Bisulfit enthaltendes Reagenz;
(b) Primerolionukleotide zur Herstellung definierter Amplifikate;
(c) zwei Klassen von Oligonukleotiden, die an einer Festphase immobilisiert sind; wobei
- die Oligonukleoide der ersten Klasse die Sequenz 5'-CG-3' umfassen, und wobei
- die Oligonukleotide der zweiten Klasse die Sequenz 5'-TG-3' und/oder die Sequenz 5-CA-3' umfassen; und
(d) eine Anleitung zur Durchführung des Verfahrens.

## Claims

1. A method for the detection of the degree of methylation of a specific cytosine in the sequence context 5'-CpG-3' of a genomic DNA sample, is hereby **characterized in that**
a) the genomic DNA is chemically treated, whereby the cytosine bases are converted to uracil, but not the 5-methylcytosine bases;
b) selected fragments, which contain said specific cytosine, are amplified, whereby the amplified products are given a detectable label;
c) the amplified products are hybridized to two classes of oligonucleotides and/or PNA oligomers, each of which class has one member of that said specific cytosine;
d) the extent of hybridization of the amplified products to the two classes of oligonucleotides and/or PNA oligomers is determined by detection of the label of the amplified products;
e) a conclusion is made on the extent of methylation of said specific cytosine in the genomic DNA sample from the ratio of the labels detected for the two classes of oligonucleotides and/or PNA oligomers as a consequence of the hybridization.

2. The method according to claim 1, further **characterized in that** a hybridization of the amplified products is conducted in step c) on two classes of oligomers (oligonucleotides and/or PNA oligomers), each of which class has one member of that said specific cytosine, whereby the oligomers of the first class preferably hybridize to the sequence which arises after the chemical treatment of the genomic DNA, if said specific cytosine was present in the methylated state in the genomic DNA and whereby the oligomers of the second class preferably hybridize to the sequence which arises after the chemical treatment of the genomic DNA, if said specific cytosine was present in the unmethylated state in the genomic DNA.

3. The method according to claim 1, further **characterized in that** a hybridization of the amplified products is conducted in step c) on two classes of oligomers (oligonucleotides and/or PNA oligomers), each of which class has one member of that said specific cytosine, whereby the oligomers of the first class preferably hybridize to the sequence which arises after the chemical treatment of the genomic DNA, if said specific cytosine was present in the methylated state in the genomic DNA and less preferably hybridize to the sequence which arises after the chemical treatment of the genomic DNA, if said specific cytosine was present in the unmethylated state in the genomic DNA, and whereby the oligomers of the second class hybridize to the amplified product to be investigated essentially independently of the degree of methylation of said specific cytosine in the genomic DNA.

4. The method according to claim 1, further **characterized in that** a hybridization of the amplified products is conducted in step c) on two classes of oligomers (oligonucleotides and/or PNA oligomers), each of which class has one member of that said specific cytosine, whereby the oligomers of the first class preferably hybridize to the sequence which arises after the chemical treatment of the genomic DNA, if said specific cytosine was present in the unmethylated state in the genomic DNA and less preferably hybridize to the sequence which arises after the chemical treatment of the genomic DNA, if said specific cytosine was present in the methylated state in the genomic DNA, and whereby the oligomers of the second class hybridize to the amplified product to be investigated essentially independently of the degree of methylation of said specific cytosine in the genomic DNA.

5. The method according to one of claims 1 to 4, further **characterized in that** the method is conducted not only with the genomic DNA sample, but also logically with standard DNA in which it is known whether the cytosine at said specific position is present in methylated or unmethylated state, whereby the ratios of the labels detected on the two classes of oligonucleotides, which are measured each time with the unmethylated standard DNA according to claim 1, serve as a calibration value for a degree of methylation of 0, and correspondingly, the ratios of the labels detected on the two classes of oligonucleotides, which are measured each time with the methylated standard DNA according to claim 1, serve as a calibration value for a degree of methylation of 1, and these calibration values are used for the determination of the degree of methylation of the genomic DNA sample.

6. The method according to claim 5, further **characterized in that** additional known standard DNA samples, each of which has any known degree of methylation of said specific cytosine, are used for calibration.

7. The method according to one of claims 5 or 6, further **characterized in that** the DNAs used as the standard are each labeled differently and the sample (the amplified product from a genomic DNA prepared according to claim 1) is provided in turn with a label that is different therefrom.

8. The method according to one of claims 1 to 7, further **characterized in that** amplified products that are derived from different genomic DNA samples are provided with different labels.

9. The method according to one of claims 1 to 8, further **characterized in that** amplified products originating from the same genomic DNA samples are provided with different labels in order to achieve an increase of measurement accuracy by an averaging of the values obtained from different detection methods.

10. The method according to one of the preceding claims, further
**characterized in that** said labels are fluorescent labels.

11. The method according to one of the preceding claims, further **characterized in that** the chemical treatment is conducted with a solution of a bisulfite (= hydrogen sulfite, disulfite).

12. The method according to one of the preceding claims, further **characterized in that** said label is detected by its chemiluminescence, its UV absorption or fluorescence polarization.

13. The method according to one of claims 1 to 11, further **characterized in that** the labels are radionuclides.

14. The method according to one of claims 1 to 11, further **characterized in that** the labels are removable mass labels, which are detected in a mass spectrometer.

15. The method according to one of claims 1 to 11, further **characterized in that** the PCR products as a whole or their characteristic fragments are detected in the mass spectrometer and thus are clearly **characterized by** their mass.

16. The method according to one of the preceding claims, further **characterized in that** the oligomers (oligonucleotides and/or PNA oligomers) of one class contain the sequence 5'-CG-3'.

17. The method according to one of the preceding claims, further **characterized in that** the oligomers (oligonucleotides and/or PNA oligomers) of one class contain the sequence 5'-TG-3' or the sequence 5'-CA-3'.

18. The method according to claims 16 or 17, further **characterized in that** the oligomers (oligonucleotides and/or PNA oligomers) of the first class contain the sequence 5'-CG-3' and the oligomers of the second class contain the sequence 5'-TG-3' or the sequence 5'-CA-3'.

19. The method according to one of the preceding claims, further **characterized in that** the oligomers of the first and the second classes are immobilized on a common solid phase.

20. The method according to claim 11, further **characterized in that** the oligonucleotides are arranged on a planar solid phase in a rectangular or hexagonal grid and the site of specific oligonucleotides on the solid phase is correlated with their respective sequence.

21. The method according to claims 1-18, further **characterized in that** the oligomers of the first and second classes are immobilized on beads, which are coded with a set of separately detectable labels.

22. The method according to one of the preceding claims, further **characterized in that** step b) of claim 1 is conducted in two sub-steps as follows:
a) a PCR pre-amplification with at least one pair of primers of different sequence which hybridize nonspecifically to a DNA sample pretreated according to claim 1 and thus produce more than one amplified product in the PCR step;
b) a PCR amplification of the product formed in the pre-amplification, with primers of different sequence, which are each identical or inversely complementary to a segment of the DNA sample [(+) strand or (-) strand] that has been pretreated according to claim 1, and hybridize specifically to the DNA to be amplified.

23. The method according to one of the preceding claims, further
**characterized in that** the amplification of several DNA segments is conducted in one reaction vessel.

24. The method according to one of the preceding claims, further **characterized in that** a heat-stable DNA polymerase is used for the amplification.

25. The method according to one of the preceding claims, further **characterized in that** the primer oligonucleotides used for the amplification contain either only the bases T, A and C or the bases T, A and G.

26. The method according to one of the preceding claims, further **characterized in that** at least 10 CpG positions in different sequence context are analyzed simultaneously.

27. The method according to one of the preceding claims, further **characterized in that** at least 50 CpG positions in different sequence context are analyzed simultaneously.

28. The method according to one of the preceding claims, further **characterized in that** at least 100 CpG positions in different sequence context are analyzed simultaneously.

29. The method according to one of the preceding claims, further **characterized in that** at least 500 CpG positions in different sequence context are analyzed simultaneously.

30. The method according to one of the preceding claims, further **characterized in that** at least 1000 CpG positions in different sequence context are analyzed simultaneously.

31. The method according to one of the preceding claims, whereby the genomic DNA sample has been obtained from cell lines, blood, sputum, stool, urine, cerebrospinal fluid, tissue embedded in paraffin, for example, tissue from eyes, intestine, kidney, brain, heart, prostate, lung, breast or liver, histological slides or all other possible combinations thereof.

32. Use of a method according to one of the preceding claims for the diagnosis and/or prognosis of adverse events for patients or individuals, whereby these adverse events belong to at least one of the following categories: undesired drug interactions; cancer diseases; CNS malfunctions, damage or disease; symptoms of aggression or behavioral disturbances; clinical, psychological consequences of brain damage; psychotic disturbances and personality disorders; dementia and/or associated syndromes; cardiovascular disease, malfunction and damage; malfunction, damage or disease of the gastrointestinal tract; malfunction, damage or disease of the respiratory system; lesion, inflammation, infection, immunity and/or convalescence; malfunction, damage or disease of the body as an abnormality in the development process; malfunction, damage or disease of the skin, the muscles, the connective tissue or the bones; endocrine and metabolic malfunction, damage or disease; headaches or sexual malfunctions.

33. Use of a method according to one of the preceding claims for the differentiation of cell types or tissues or for investigation of cell differentiation.

34. A kit, for conducting a method according to one of the preceding claims, comprising
(a) a reagent containing bisulfite;
(b) primer oligonucleotides for preparing the selected amplified products;
(c) two classes of oligonucleotides immobilized on a solid phase; whereas
- the oligonucleotides of the first class contain the sequence 5'-CG-3', and whereas
- the oligonucleotides of the second class contain the sequence 5'-TG-3' and/or the sequence 5'-CA-3'; and
(d) an instruction for conducting the method.

## Revendications

1. Procédé de détection du taux de méthylation d'une cytosine déterminée dans le contexte de séquence 5'-CpG-3' d'une sonde d'ADN génomique, **caractérisé en ce que** :
a) on traite chimiquement l'ADN génomique, où l'on transforme les bases cytosine en uracile, mais pas les bases 5-méthylcytosine ;
b) on amplifie un fragment défini, qui contient la cytosine déterminée mentionnée, où le produit d'amplification contient un marqueur détectable ;
c) on hybride le produit d'amplification sur deux classes d'oligonucléotides et/ou d'oligomères PNA avec chaque fois un membre pour la cytosine déterminée mentionnée ;
d) on détermine la mesure de l'hybridation du produit d'amplification sur les deux classes d'oligonucléotides et/ou d'oligomères PNA par détection du marqueur du produit d'amplification ;
e) on conclut le taux de méthylation de la cytosine déterminée mentionnée dans l'échantillon d'ADN génomique à partir du rapport du marquage détecté sur les deux classes d'oligonucléotides et/ou d'oligomères PNA suite à l'hybridation.

2. Procédé selon la revendication 1, **caractérisé en ce qu'**à l'étape c), on réalise une hybridation du produit d'amplification sur deux classes d'oligomères (oligonucléotides et/ou oligomères PNA) avec chaque fois un membre pour la cytosine déterminée mentionnée, où les oligomères de la première classe s'hybrident de préférence à la séquence qui résulte du traitement chimique de l'ADN génomique, lorsque la cytosine déterminée mentionnée existe à l'état méthylé dans l'ADN génomique et où les oligomères de la deuxième classe s'hybrident de préférence à la séquence qui résulte du traitement chimique de l'ADN génomique, lorsque la cytosine déterminée mentionnée existe à l'état non méthylé dans l'ADN génomique.

3. Procédé selon la revendication 1, **caractérisé en ce qu'**à l'étape c), on réalise une hybridation du produit d'amplification sur deux classes d'oligomères (oligonucléotides et/ou oligomères PNA) avec chaque fois un membre pour la cytosine déterminée mentionnée, où les oligomères de la première classe s'hybrident de préférence à la séquence qui résulte du traitement chimique de l'ADN génomique, lorsque la cytosine déterminée mentionnée existe à l'état méthylé dans l'ADN génomique et de manière moins préférée, à la séquence qui résulte du traitement chimique de l'ADN génomique, lorsque la cytosine déterminée mentionnée existe à l'état non méthylé dans l'ADN génomique, et où les oligomères de la deuxième classe s'hybrident au produit d'amplification à examiner, essentiellement indépendamment du taux de méthylation de la cytosine déterminée mentionnée dans l'ADN génomique.

4. Procédé selon la revendication 1, **caractérisé en ce qu'**à l'étape c), on réalise une hybridation du produit d'amplification sur deux classes d'oligomères (oligonucléotides et/ou oligomères PNA) avec chaque fois un membre pour la cytosine déterminée mentionnée, où les oligomères de la première classe s'hybrident de préférence à la séquence qui résulte du traitement chimique de l'ADN génomique, lorsque la cytosine déterminée mentionnée existe à l'état non méthylé dans l'ADN génomique et de manière moins préférée, à la séquence qui résulte du traitement chimique de l'ADN génomique, lorsque la cytosine déterminée mentionnée existe à l'état méthylé dans l'ADN génomique, et où les oligomères de la deuxième classe s'hybrident au produit d'amplification à examiner, essentiellement indépendamment du taux de méthylation de la cytosine déterminée mentionnée dans l'ADN génomique.

5. Procédé selon l'une des revendications 1 à 4, **caractérisé en ce que** le procédé est réalisé non seulement avec l'échantillon d'ADN génomique, mais aussi, en substance, avec l'ADN standard présent de manière connue à l'état méthylé ou non méthylé en la position de la cytosine déterminée mentionnée, où les rapports, mesurés chaque fois avec l'ADN standard non méthylé selon la revendication 1, du marquage détecté sur les deux classes d'oligonucléotides servent de valeur étalon pour un taux de méthylation de 0 et de manière correspondante, les rapports, mesurés chaque fois avec l'ADN standard méthylé selon la revendication 1, du marquage détecté sur les deux classes d'oligonucléotides servent de valeur étalon pour un taux de méthylation de 1 et on met en oeuvre ces valeurs étalons pour la détermination du taux méthylation des échantillons d'ADN génomique.

6. Procédé selon la revendication 5, **caractérisé en ce que** l'on utilise d'autres échantillons connus d'ADN standard pour l'étalonnage, lesquels présentent chaque fois un taux de méthylation connu quelconque de la cytosine déterminée mentionnée.

7. Procédé selon la revendication 5 ou 6, **caractérisé en ce que** les ADN utilisés comme standard sont à chaque fois marqués différemment et l'échantillon (le produit d'amplification préparé à partir d'un ADN génomique correspondant à la revendication 1) est muni à nouveau d'un marquage différent de ceux-ci.

8. Procédé selon l'une des revendications 1 à 7, **caractérisé en ce que** les produits d'amplification, résultant de différents échantillons d'ADN génomique, sont munis de marquages différents.

9. Procédé selon l'une des revendications 1 à 8, **caractérisé en ce que** les produits d'amplification, résultant du même échantillon d'ADN génomique, sont munis de marquages différents, pour atteindre une augmentation de la précision de mesure par moyenne des valeurs obtenues à partir des différents procédés de détection.

10. Procédé selon l'une des revendications précédentes, **caractérisé en ce que** les marquages mentionnés sont des marquages de fluorescence.

11. Procédé selon l'une des revendications précédentes, **caractérisé en ce que** le traitement chimique est réalisé avec une solution d'un bisulfite (= hydrogénosulfite, disulfite).

12. Procédé selon l'une des revendications précédentes, **caractérisé en ce que** l'on détecte le marquage mentionné par sa chimioluminescence, son absorption UV ou sa polarisation de fluorescence.

13. Procédé selon l'une des revendications 1 à 11, **caractérisé en ce que** les marquages sont des radionucléides.

14. Procédé selon l'une des revendications 1 à 11, **caractérisé en ce que** les marquages sont des marquages de masse éliminables, qui sont détectés dans un spectromètre de masse.

15. Procédé selon l'une des revendications 1 à 11, **caractérisé en ce que** l'on détecte les produits PCR globalement ou leurs fragments caractéristiques dans le spectromètre de masse et ainsi, ils sont **caractérisés** de manière non ambiguë par leur masse.

16. Procédé selon l'une des revendications précédentes, **caractérisé en ce que** les oligomères (oligonucléotides et/ou oligomères PNA) d'une classe comprennent la séquence 5'-CG-3'.

17. Procédé selon l'une des revendications précédentes, **caractérisé en ce que** les oligomères (oligonucléotides et/ou oligomères PNA) d'une classe comprennent la séquence 5'-TG-3' ou la séquence 5'-CA-3'.

18. Procédé selon les revendications 16 et 17, **caractérisé en ce que** les oligomères (oligonucléotides et/ou oligomères PNA) de la première classe comprennent la séquence 5'-CG-3' et les oligomères de la deuxième classe comprennent la séquence 5'-TG-3' ou la séquence 5'-CA-3'.

19. Procédé selon l'une des revendications précédentes, **caractérisé en ce que** les oligomères des première et deuxième classes sont immobilisés sur une phase solide commune.

20. Procédé selon la revendication 11, **caractérisé en ce que** les oligonucléotides sont agencés sur une phase solide plane dans un cadre carré ou hexagonal et l'emplacement des oligonucléotides déterminés sur la phase solide est corrélé à leur séquence respective.

21. Procédé selon l'une des revendications 1 à 18, **caractérisé en ce que** les oligomères des première et deuxième classes sont immobilisés sur des billes, qui sont codées avec un jeu de marquages détectables séparément.

22. Procédé selon l'une des revendications précédentes, **caractérisé en ce que** l'on réalise l'étape b) de la revendication 1 en deux étapes partielles, comme suit :
a) pré-amplification PCR avec au moins une paire d'amorces de séquences différentes, qui s'hybrident de manière non spécifique sur un échantillon d'ADN pré-traité selon la revendication 1 et donnent dans l'étape PCR, plus d'un produit d'amplification ;
b) amplification PCR du produit formé dans la pré-amplification, avec des amorces de séquences différentes, qui sont identiques ou inverses et complémentaires, chaque fois à un segment de l'échantillon d'ADN [brin (+) ou brin (-)] pré-traité selon la revendication 1 et qui s'hybrident spécifiquement à l'ADN à amplifier.

23. Procédé selon l'une des revendications précédentes, **caractérisé en ce que** l'amplification de plusieurs segments d'ADN est réalisée dans un récipient de réaction.

24. Procédé selon l'une des revendications précédentes, **caractérisé en ce que** pour l'amplification, on utilise une ADN polymérase stable à la chaleur.

25. Procédé selon l'une des revendications précédentes, **caractérisé en ce que** pour l'amplification, les oligonucléotides d'amorce utilisés contiennent seulement les bases T, A et C ou seulement les bases T, A et G.

26. Procédé selon l'une des revendications précédentes, **caractérisé en ce que** l'on analyse simultanément au moins 10 positions CpG dans différents contextes de séquence.

27. Procédé selon l'une des revendications précédentes, **caractérisé en ce que** l'on analyse simultanément au moins 50 positions CpG dans différents contextes de séquence.

28. Procédé selon l'une des revendications précédentes, **caractérisé en ce que** l'on analyse simultanément au moins 100 positions CpG dans différents contextes de séquence.

29. Procédé selon l'une des revendications précédentes, **caractérisé en ce que** l'on analyse simultanément au moins 500 positions CpG dans différents contextes de séquence.

30. Procédé selon l'une des revendications précédentes, **caractérisé en ce que** l'on analyse simultanément au moins 1000 positions CpG dans différents contextes de séquence.

31. Procédé selon l'une des revendications précédentes, dans lequel les échantillons d'ADN génomique sont obtenus de lignées cellulaires, de sang, de pus, de selles, d'urine, de fluide cérébral-moelle épinière, de tissu piégé dans la paraffine, par exemple du tissu d'oeil, d'intestin, de rein, de cerveau, de coeur, de prostate, de poumon, de sein ou de foie, de supports d'objets histologiques ou de toutes les combinaisons possibles de ceux-ci.

32. Utilisation d'un procédé selon l'une des revendications précédentes, pour le diagnostic et/ou le pronostic d'événements défavorables chez des patients ou des individus, les événements défavorables appartenant à au moins une des catégories suivantes : effets médicamenteux indésirables ; cancers ; dysfonctionnement, lésions ou maladies du SNC ; symptômes d'agression ou troubles du comportement ; conséquences cliniques, psychologiques de lésions au cerveau ; troubles psychotiques et troubles de la personnalité ; démence et/ou syndrome associé ; maladie, dysfonctionnement et lésion cardiovasculaires ; dysfonctionnement, lésion ou maladie du tractus gastro-intestinal ; dysfonctionnement, lésion ou maladie du système respiratoire ; blessure, inflammation, infection, immunité et/ou convalescence ; dysfonctionnement, lésion ou maladie du corps par rapport au processus de développement ; dysfonctionnement, lésion ou maladie de la peau, des muscles, des muqueuses ou des os ; dysfonctionnement, lésion ou maladie endocrine et métabolique ; migraines ou dysfonctionnement sexuel.

33. Utilisation d'un procédé selon l'une des revendications précédentes, pour différencier des types de cellules ou des tissus ou pour étudier la différenciation cellulaire.

34. Kit pour réaliser un procédé selon l'une des revendications précédentes, comprenant :
(a) un réactif contenant un bisulfite ;
(b) des oligonucléotides d'amorce pour la préparation d'un produit d'amplification défini ;
(c) deux classes d'oligonucléotides, qui sont immobilisés sur une phase solide, où :
- les oligonucléotides de la première classe comprennent la séquence 5'-CG-3', et
- les oligonucléotides de la deuxième classe comprennent la séquence 5'-TG-3' et/ou la séquence 5'-CA-3', et
(d) les directives pour réaliser le procédé.


### Sequence listing

<110> Epigenomics AG
<120> Verfahren zur Bestimmung des Methylierungsgrades von bestimmten Cytosinen in genomischer DNA im sequenzkontext 5'-CpG-3'
<160> 11
<210> 1
   <211> 24
   <212> DNA
   <213> Artificial Sequence
<220>
   <223> Oligonukleotid
<400> 1
   gctctatggt cttgtctaac cgta 24
<210> 2
   <211> 18
   <212> DNA
   <213> Artificial Sequence
<220>
   <223> Oligonukleotid
<400> 2
   aggtggtggt ggcggtgg 18
<210> 3
   <211> 28
   <212> DNA
   <213> Artificial Sequence
<220>
   <223> Oligonukleotid
<400> 3
   atggttttgt ttaatygtag agttgttt 28
<210> 4
   <211> 27
   <212> DNA
   <213> Artificial Sequence
<220>
   <223> oligonukleotid
<400> 4
   taaacccraa aaaaaaaaac ccaatat 27
<210> 5
   <211> 18
   <212> DNA
   <213> Artificial Sequence
<220>
   <223> oligonukleotid
<400> 5
   ctactcaacg aaaacaaa 18
<210> 6
   <211> 18
   <212> DNA
   <213> Artificial sequence
<220>
   <223> oligonukleotid
<400> 6
   ctactcaaca aaaacaaa 18
<210> 7
   <211> 20
   <212> DNA
   <213> Artificial sequence
<220>
   <223> Oligonukleotid
<400> 7
   agcaacacct ccatgcactg 20
<210> 8
   <211> 21
   <212> DNA
   <213> Artificial Sequence
<220>
   <223> oligonukleotid
<400> 8
   ttgattggac agcttgaatg c 21
<210> 9
   <211> 13
   <212> DNA
   <213> Artificial Sequence
<220>
   <223> Oligonukleotid
<400> 9
   gaagagcgga cag 13
<210> 10
   <211> 18
   <212> DNA
   <213> Artificial Sequence
<220>
   <223> oligonukleotid
<400> 10
   attaatagcg ttttggtt 18
<210> 11
   <211> 18
   <212> DNA
   <213> Artificial Sequence
<220>
   <223> Oligonukleotid
<400> 11
   attaatagtg ttttggtt 18